# EUROPEAN PATENT APPLICATION

(11) **EP 3 747 456 A1**
(43) Date of publication of application: **09.12.2020**
(21) Application number: 19746998.4
(22) Date of filing: 31.01.2019
(51) Int. Cl.: A61K 38/16, A61K 39/395, A61P 37/06, A61P 37/08, A61P 43/00, G01N 33/53, G01N 33/564, C07K 14/725, C07K 16/00, C07K 19/00, C12N 15/12, C12N 15/13

(54) **METHOD FOR REGULATING ANTIGEN-SPECIFIC MHC EXPRESSION**

(30) Priority: 31.01.2018 JP 2018015908
(71) Applicant: Tohoku University, Sendai-shi, Miyagi 980-8577 (JP)
(72) Inventor: OGASAWARA Koetsu, Sendai-shi, Miyagi 980-8577 (JP)
(74) Representative: Mewburn Ellis LLP
(86) International application number: PCT/JP2019/003363
(87) International publication number: WO 2019/151392

(57) **Abstract**

Provided is a method for treating and diagnosing an autoimmune disease, organ transplant rejection and allergic disease by using a method of regulating antigen-specific MHC expression. An agent for decreasing T-cell function for suppressing an autoimmune disease, organ transplant rejection or allergic disease, comprising a T-cell receptor or chimeric protein being a fusion protein of a T-cell receptor variable region recognizing a complex of an autoimmune disease-specific antigen and an MHC molecule, a complex of a specific antigen causing an organ transplant rejection and an MHC molecule, or a complex of an allergic disease-specific antigen and an MHC molecule and an immunoglobulin Fc region, as an active ingredient; and regulating expression of the MHC molecule.

## Description

### Technical Field

The present invention relates to treatment or diagnosis of an autoimmune disease, organ transplant rejection or allergic disease.

### Background Art

An autoimmune disease is a disorder caused by abnormality in response of immune cells and reported to have a strong correlation with the type of MHC. In organ transplant, attempts have been made to prevent a rejection by partly matching MHC types. As mentioned, MHC is an important molecule in autoimmune disease and organ transplant.

MHC types are known as white blood types and used as a means for identifying individuals. MHC types are roughly divided into class I and class II, present an antigen peptide thereon to form an MHC complex.

Immune response occurs when a T-cell receptor (TCR) on T cells binds to an MHC complex and activates T cells in an antigen-peptide specific manner. The T-cell receptor plays an important role in the action of T cells. In recent years, a method has been developed for identifying a disease-specific T-cell receptor by analyzing a T-cell receptor repertoire of a T-cell receptor of a patient having a disease. Also, a method of using a protein having a T-cell receptor fragment has been reported.

As a method for regulating the reaction of T cells, controlling the reaction of a T cell itself has been investigated in various ways; however, considering that immune response of a T cell occurs when a T-cell receptor binds to a MHC complex, it would be sufficient if expression of MHC can be specifically regulated. Conventionally, the expression has been technically regulated by use of an MHC-specific antibody. For example, mouse MHC class I molecules, H-2K, D and L are known, against which antibodies have been prepared. The anti-MHC antibodies are known to induce down modulation of MHC molecules (Patent Literature 1). However, these antibodies do not have specificity to antigen peptides. Because of this, they presumably have severe side effects.

### Citation List

### Patent Literature

[Patent Literature 1] JP Patent Publication (Kokai) No. 2017-128585

### Summary of Invention

### Technical Problem

An object of the present invention is to provide a method for treating or diagnosing an autoimmune disease, organ transplant rejection or allergic disease by regulating expression MHC (major histocompatibility complex) on the surface of cells in an antigen-peptide specific manner by use of the recognition mechanism of a T-cell receptor (TCR).

An MHC molecule can present an antigen peptide; however, the antibody to MHC binds only to MHC and rarely recognizes an antigen peptide as well as an MHC molecule and binds them. An antibody capable of recognizing a complex of an OVA peptide and an MHC complex is an exception. Usually, it takes about 6 months to prepare an antibody capable of recognizing an MHC complex, which varies depending on an antigen peptide. Likewise, preparation of the antibody is not easy. Accordingly, a problem is that preparation of a molecule for regulating expression of MHC on a cell surface, which varies depending on an antigen peptide, is difficult.

Then, the present inventors thought that, since a T-cell receptor recognizes an antigen peptide and an MHC complex, the T-cell receptor might be used like an antibody. They also thought that, if expression of an antigen peptide and an MHC complex on a cell surface can be reduced through down modulation by a T-cell receptor chimeric protein, a lead compound of drugs for various diseases can be obtained.

### Solution to Problem

Conventionally, a monoclonal antibody has been used for regulating expression of a cell-surface molecule. For example, to mouse MHC class I molecules, H-2K, D and L, against which antibodies have been respectively prepared. An antigen peptide can be presented by an MHC molecule; however, there is little in the antibodies to the MHC (molecule), which recognize an antigen peptide and the MHC molecule and then bind them. In addition, it is still difficult to prepare an antibody capable of recognizing an MHC complex in an antigen peptide dependent manner. In short, it has been difficult to prepare a molecule for regulating expression of MHC in an antigen peptide dependent manner.

Presently, an antigen-peptide specific T-cell receptor has been cloned by a T-cell receptor (TCR) analysis technology (International Publication No. WO2016/136716). Based on the information, a T-cell receptor chimeric protein came to be successfully produced.

The T-cell receptor chimeric protein binds to MHC and a peptide complex in an antigen-peptide specific manner and induces down modulation of MHC to reduce the expression.

An autoimmune disease has a strong correlation with an MHC type and a specific MHC type is frequently expressed. Thus, the T-cell receptor chimeric protein, which induces down modulation of an antigen peptide and an MHC complex and reduces expression, can be used in developing a drug for an autoimmune disease and an organ transplant rejection.

As described above, the present inventors found a method for regulating cell-surface expression of an MHC complex in an antigen-peptide specific manner by using a TCR chimeric protein. Owing to the method, it is expected that T cell response can be suppressed, resulting in suppression of onset of an autoimmune disease, organ transplant rejection and allergic disease.

More specifically, the present invention is as follows.
[1] An agent for down-modulating MHC molecular complex
   comprising, as an active ingredient, a T-cell receptor chimeric protein being a fusion protein of a T-cell receptor variable region recognizing a complex of an autoimmune disease-specific antigen and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex, in an antigen specific manner.
[2] An agent for down-modulating MHC molecular complex comprising, as an active ingredient, a T-cell receptor chimeric protein being a fusion protein of a T-cell receptor variable region recognizing a complex of a donor-derived antigen specific to an organ transplant rejection and an MHC molecule and an immunoglobulin Fc region, wherein the agent binds to a complex of the donor-derived specific antigen and the MHC molecule on an antigen-presenting cell or a target cell recognized by a pathogenic T cell to reduce the expression of the MHC molecular complex, in an antigen specific manner.
[3] An agent for down-modulating MHC molecular complex comprising, as an active ingredient, a T-cell receptor chimeric protein being a fusion protein of a T-cell receptor variable region recognizing a complex of a specific antigen to an allergic disease and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex, in an antigen specific manner.
[4] The agent for down-modulating MHC molecular complex according to any one of [1] to [3], wherein the T-cell receptor chimeric protein comprises a variable region, whole of CDR3 and J region of a T-cell receptor.
[5] The gent for down-modulating MHC molecular complex according to any one of [1] to [4], wherein the T-cell receptor variable region is α chain and/or β chain of the T-cell receptor.
[6] The gent for down-modulating MHC molecular complex according to any one of [1] to [5], wherein the immunoglobulin Fc region is an Fc region of IgG.
[7] The gent for down-modulating MHC molecular complex according to any one of [1] to [6], being a dimer of two fusion proteins of the T-cell receptor variable region and the immunoglobulin Fc region wherein the two proteins are bonded to each other by disulfide bond.
[8] The gent for down-modulating MHC molecular complex according to any one of [1] to [7], wherein the T-cell receptor binds to the complex of the antigen and the MHC molecule.
[9] The gent for down-modulating MHC molecular complex according to any one of [1] to [8], wherein the MHC molecule is a classical MHC molecule or a non-classical MHC molecule.
[10] An agent for decreasing T-cell function causing an autoimmune disease, comprising, as an active ingredient, a T-cell receptor chimeric protein being a fusion protein of a T-cell receptor variable region recognizing a complex of an autoimmune disease-specific antigen and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex in order to avoid recognition by the pathogenic T cell.
[11] An agent for decreasing T-cell function for decreasing T-cell function causing organ transplant rejection, comprising, as an active ingredient, a T-cell receptor chimeric protein being a fusion protein of a T-cell receptor variable region recognizing a complex of a donor-derived antigen specific to an organ transplant rejection and an MHC molecule and an immunoglobulin Fc region, wherein the agent binds to a complex of the donor-derived specific antigen and the MHC molecule on an antigen-presenting cell or a target cell recognized by a pathogenic T cell to reduce the expression of the MHC molecular complex in order to avoid recognition by the pathogenic T cell.
[12] An agent for decreasing T-cell function causing an allergic disease, comprising, as an active ingredient, a T-cell receptor chimeric protein being a fusion protein of a T-cell receptor variable region recognizing a complex of an allergic disease-specific antigen and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex in order to avoid recognition by the pathogenic T cell.
[13] The agent for decreasing T-cell function according to any one of [10] to [12], wherein the T-cell receptor chimeric protein comprises a variable region, whole of CDR3 and J region of a T-cell receptor.
[14] The agent for decreasing T-cell function according to any one of [10] to [13], wherein the T-cell receptor variable region is α chain and/or β chain of the T-cell receptor.
[15] The agent for decreasing T-cell function according to any one of [10] to [14], wherein the immunoglobulin Fc region is an Fc region of IgG.
[16] The agent for decreasing T-cell function according to any one of [10] to [15], wherein the agent is a dimer of two fusion proteins of the T-cell receptor variable region and the immunoglobulin Fc region, wherein the two proteins are bonded to each other by disulfide bond.
[17] The agent for decreasing T-cell function according to any one of [10] to [16], wherein the T-cell receptor binds to the complex of the antigen and the MHC molecule.
[18] The agent for decreasing T-cell function according to any one of [10] to [17], wherein the MHC molecule is a classical MHC molecule or a non-classical MHC molecule.
[19] A therapeutic agent for an autoimmune disease, comprising the agent for down-modulating the MHC molecular complex according to any one of [1] and [4] to [9].
[20] An agent for suppressing an organ transplant rejection, containing the agent for down-modulating the MHC molecular complex agent according to any one of [2] and [4] to [9].
[21] A therapeutic agent for an allergic disease, comprising the agent for down-modulating the MHC molecular complex according to any one of [3] to [9].
[22] A therapeutic agent for an autoimmune disease, comprising the agent for decreasing T-cell function according to any one of [10] and [13] to [18].
[23] An agent for suppressing an organ transplant rejection, comprising the agent for decreasing T-cell function according to any one of [11] and [13] to [18].
[24] A therapeutic agent for an allergic disease, comprising the agent for decreasing T-cell function according to any one of [12] to [18].
[25] A method for detecting an autoimmune disease, comprising the steps of:
   bringing a labeled T-cell receptor chimeric protein being a fusion protein of a T-cell receptor variable region recognizing a complex of an autoimmune disease-specific antigen and an MHC molecule and an immunoglobulin Fc region, into contact with cells taken from a biological sample of a test subject; and
   determining that a target cell is present in the test subject and the test subject has an autoimmune disease if the T-cell receptor chimeric protein binds to a cell taken from the biological sample of the test subject.
[26] The method for detecting an autoimmune disease according to [25], wherein the MHC molecule is a classical MHC molecule or a non-classical MHC molecule.
[27] A reagent for detecting an autoimmune disease, comprising a labeled T-cell receptor chimeric protein being a fusion protein of a T-cell receptor variable region recognizing a complex of an autoimmune disease-specific antigen and an MHC molecule and an immunoglobulin Fc region.
[28] The reagent for detecting an autoimmune disease, according to [27], wherein the MHC molecule is a classical MHC molecule or a non-classical MHC molecule.
[29] A method for detecting a rejection causing organ transplant rejection, comprising the steps of:
   bringing a labeled T-cell receptor chimeric protein being a fusion protein of a T-cell receptor variable region recognizing a complex of a donor-derived antigen specific to organ transplant rejection and an MHC molecule and an immunoglobulin Fc region, into contact with cells taken from a biological sample of a test subject; and
   determining that a target cell is present in the test subject and a rejection occurs in the test subject if the T-cell receptor chimeric protein binds to a cell taken from the biological sample of the test subject.
[30] The method for detecting a rejection according to [29], wherein the MHC molecule is a classical MHC molecule or a non-classical MHC molecule.
[31] A reagent for detecting a rejection, comprising a labelled T-cell receptor chimeric protein being a fusion protein of a T-cell receptor variable region recognizing a complex of an organ transplant rejection-specific donor-derived antigen and the MHC molecule and an immunoglobulin Fc region.
[32] The reagent for detecting a rejection according to [31], wherein the MHC molecule is a classical MHC molecule or a non-classical MHC molecule.
[33] A method for detecting an allergic disease, comprising the steps of:
   bringing a labeled T-cell receptor chimeric protein being a fusion protein of a T-cell receptor variable region recognizing a complex of an allergic disease-specific antigen and an MHC molecule and an immunoglobulin Fc region, into contact with cells taken from a biological sample of a test subject; and
   determining that a target cell is present in the test subject and the test subject has an allergic disease if the T-cell receptor chimeric protein binds to a cell taken from the biological sample of the test subject.
[34] The method for detecting an allergic disease according to [33], wherein the MHC molecule is a classical MHC molecule or a non-classical MHC molecule.
[35] A reagent for detecting an allergic disease, comprising a labeled T-cell receptor chimeric protein being a fusion protein of a T-cell receptor variable region recognizing a complex of an allergic disease-specific antigen and an MHC molecule and an immunoglobulin Fc region.
[36] The reagent for detecting an allergic disease according to [35], wherein the MHC molecule is a classical MHC molecule or a non-classical MHC molecule.

The specification incorporates the contents disclosed in JP Patent Application No. 2018-015908, based on which the priority of the present application is claimed.

### Advantageous Effects of Invention

T-cell receptor (TCR) chimeric protein binds to a complex of an MHC class I molecule or class II molecule and an antigen peptide, induces down modulation of an MHC molecule presenting an antigen of an antigen-presenting cell or a disease target cell to reduce the expression thereof. The down modulation of an MHC molecule herein refers to suppressively regulating expression of the MHC molecule to reduce the expression. As a result, reactivity of a pathogenic T cell to the antigen presenting cell changes and the function of the T cell decreases. It is possible to suppress or treat an autoimmune disease, organ transplant rejection or allergic disease by decreasing the function of T cells.

The T-cell receptor chimeric protein, which can bind to a disease target cell expressing an MHC molecule, can be used for detection of the disease target cell.

Up to present, it has not been reported that the T-cell receptor chimeric protein induces down modulation of an antigen peptide-MHC complex. The T-cell receptor chimeric protein can be prepared simply in about two weeks based on the genetic information obtained. The preparation time can be drastically reduced compared to that of a conventional monoclonal antibody (about 6 months from immunization with an antigen to completion of screening). In the case of a monoclonal antibody, confirmation of an epitope is required (i.e., a recognition site); however, in the case of a T-cell receptor, which is a ligand of MHC, confirmation of a recognition site can be easily made just by changing an antigen peptide.

Furthermore, in the case of using the same MHC in combination with a different antigen peptide, a T-cell receptor chimeric protein can be easily prepared.

### Brief Description of Drawings

[Figure 1] Figure 1 shows the structure of T-cell receptor chimeric protein (TCR-IgFc).
[Figure 2] Figure 2 schematically shows a method for producing a T-cell receptor chimeric protein (TCR-IgFc) by gene recombination technique.
[Figure 3] Figure 3 shows an experimental process of TCR repertoire analysis for OVA.
[Figure 4] Figure 4 shows TCRs of mice sensitized with OVA peptide in comparison with that of OT-1 mouse.
[Figure 5] Figure 5 shows TCR-IgFc capable of recognizing OVA257 peptide.
[Figure 6] Figure 6 shows internalization of T-cell receptor chimeric protein bound to MHC class I molecule in a cell.
[Figure 7] Figure 7 shows an experimental process for examining behavior of TCR-IgFc complex bound to an MHC molecule.
[Figure 8] Figure 8 shows that a TCR-IgFc complex bound to an MHC molecule migrates into a cell to regulate MHC on a cell surface.
[Figure 9] Figure 9 shows a method for preparing an experimental autoimmune encephalomyelitis (EAE) model mouse.
[Figure 10] Figure 10 shows specific binding of TCR-IgFc to an antigen peptide in the case of experimental autoimmune encephalomyelitis.
[Figure 11] Figure 11 shows specific binding of TCR-IgFc to an antigen peptide in *in-vitro* cultured cells.
[Figure 12] Figure 12 shows internalization of TCR-IgFc bound to an MHC class II molecule in a cell.
[Figure 13] Figure 13 shows suppression effect of TCR-IgFc in the case of experimental autoimmune encephalomyelitis.
[Figure 14] Figure 14 shows suppression effect of TCR-IgFc on pathogenic T cells in the case of experimental autoimmune encephalomyelitis.
[Figure 15] Figure 15 shows suppression effect of TCR-IgFc on pathogenic T cells in the case of experimental autoimmune encephalomyelitis.
[Figure 16] Figure 16 shows repertoire analysis of T-cell receptor α chain reacted in a mouse lymphocyte mixed culture test.
[Figure 17] Figure 17 shows repertoire analysis of T-cell receptor β chain reacted in a mouse lymphocyte mixed culture test.
[Figure 18] Figure 18 shows suppression effect of TCR-IgFc in a mouse lymphocyte mixed culture test.
[Figure 19] Figure 19 shows suppression effect of TCR-IgFc in a mouse lymphocyte mixed culture test.
[Figure 20] Figure 20 shows an experimental process of TCR-IgFc for inhibiting activated T cells in the case of metallic allergy.
[Figure 21] Figure 21 shows an inhibitory effect of TCR-IgFc on activated T cells in the case of metallic allergy.
[Figure 22] Figure 22 schematically shows an experimental process for treating experimental autoimmune encephalomyelitis with TCR-IgFc.
[Figure 23] Figure 23 schematically shows a therapeutic effect of TCR-IgFc on experimental autoimmune encephalomyelitis.
[Figure 24] Figure 24 shows the ratio of mice which get a clinical score of 3 or more until day 17 when experimental autoimmune encephalomyelitis is treated with TCR-IgFc.
[Figure 25] Figure 25 schematically shows an experimental process for treating metallic allergy with TCR-IgFc.
[Figure 26] Figure 26 shows a therapeutic effect of TCR-IgFc on metallic allergy.

### Description of Embodiments

Now, the present invention will be more specifically described.

### 1. T-cell receptor chimeric protein

### (1) Structure of T-cell receptor chimeric protein

In the method of the present invention, a T-cell receptor chimeric protein is used, which is prepared by fusing a variable region of a T-cell receptor (TCR) and an immunoglobulin Fc region. The T-cell receptor chimeric protein is also referred to as a T-cell receptor-immunoglobulin chimeric protein or a TCR-IgFc fusion protein.

The T-cell receptor chimeric protein of the present invention is a chimeric protein prepared by binding a T-cell receptor variable region (TCR V region), whole of CDR3 and J region to an IgFc portion. Alternatively, the T-cell receptor chimeric protein of the present invention may be a chimeric protein, which is prepared by binding a T-cell receptor variable region (TCR V region), whole of CDR3, J region and a part of C region to an IgFc portion. The T-cell receptor chimeric protein of the present invention can be expressed as TCR-IgFc.

As the variable region of the T-cell receptor, a variable region of a T-cell receptor, which recognizes an antigen specific to an autoimmune disease, organ transplant rejection or allergic disease, is used. A T-cell receptor variable region of the present invention recognizes a complex of an autoimmune disease-specific antigen and an MHC molecule, a complex of a donor-derived antigen (antigen peptide) specific to organ transplant rejection and an MHC molecule, or a complex of an allergic disease-specific antigen and an MHC molecule. As a result, the T-cell receptor chimeric protein of the present invention binds to a complex of a specific antigen on an antigen presenting cell or a target cell, recognized by a pathogenic T cell of an autoimmune disease and the MHC molecule, a complex of a donor-derived specific antigen on an antigen presenting cell or a target cell recognized by a pathogenic T cell of organ transplantation rejection and the MHC molecule, or a complex of a specific antigen on an antigen presenting cell or a target cell recognized by a pathogenic T cell of an allergic disease and the MHC molecule to not only cause competitive inhibition with the pathogenic T cell but also reduce the expression of the MHC molecular complex in an antigen specific manner. Consequently, the T-cell receptor chimeric protein of the present invention prevents recognition of the cells expressing an MHC molecule by T cells, thereby decreasing the function of T cells. In the present invention, MHC is not limited to classical MHC (class Ia, class II) and non-classical MHC, e.g., so-called a class Ib molecule is included. Examples of the class Ib molecule include molecules such as CD1, MR1, H2-M3 and HLA-G, which are confirmed to bind to T cells (Biochemistry, vol, 81. No.3, pp.189-199, 2009). Reduced expression of MHC molecular complex includes reduced expression of MHC gene and decreased expression of an MHC molecule on a cell surface, and is preferably, reduced expression of an MHC molecule on a cell surface.

A T-cell receptor is a dimer constituted of α and β chains, or γ chain and δ chains each formed of a variable region and a constant region. The variable region is encoded by a plurality of gene fragments: V (variable) regions (V gene fragments), D (diversity) regions and a J (joining) regions (β chain, δ chain); or V regions and J regions (α chain, γ chain). The variable region comes to have a number of repertoires through gene rearrangement. Further, the variable region has three hypervariable regions each called CDR (complementarity determining region) and further comes to have a larger number of repertoires through somatic mutations of these regions. In particular, a CDR3 region is involved in antigen specificity and sequence variations are likely to occur therein, so that it has a large sequence diversity.

The T-cell receptor variable region of the T-cell receptor chimeric protein of the present invention is constituted of α chain or β chain and may be a single chain of a fusion of α chain and β chain. In a T-cell receptor chimeric protein constituting a dimer, one of the chains may be a fusion protein of an immunoglobulin Fc region and T-cell receptor α chain; whereas, the other chain may be a fusion protein of an immunoglobulin Fc region and T-cell receptor β chain. Alternatively, a T-cell receptor variable region of the T-cell receptor chimeric protein is constituted of γ chain and δ chain and may be a single chain of a fusion of γ chain and δ chain. In the T-cell receptor chimeric protein constituting a dimer, one of the chains may be a fusion protein of an immunoglobulin Fc region and T-cell receptor γ chain; whereas, the other chain may be a fusion protein of an immunoglobulin Fc region and T-cell receptor δ chain. In a T-cell receptor variable region, α, β, γ and δ chains are each constituted of about 200 to 400 amino acids. The α, β, γ and δ chains of the T-cell receptor variable region to be used in the present invention include those of a T-cell receptor variable region whose amino acid sequences have an identity of 90% or more, 95% or more, 97% or more, or 99% or more with that of a naturally occurring T-cell receptor variable region, as calculated by use of, e.g., BLAST (Basic Local Alignment Search Tool at the National Center for Biological Information)(using, for example, default, i.e., parameters of initial setting).

T-cell receptor chimeric protein of the present invention is preferably a multimer, such as a dimer or a tetramer. Examples of the dimer variable region include a combination of α chain-α chain, a combination of α chain-β chain, and a combination of β chain-β chain. Examples of the tetramer variable region include a combination of α chain-α chain and a combination of β chain-β chain; and further include a combination of γ chain- γ chain, a combination of γ chain-δ chain and a combination of δ chain-δ chain. Examples of the tetramer variable region include a combination of γ chain-γ chain and δ chain-δ chain.

The T-cell receptor variable region may be derived from any T-cell as long as it binds to MHC. The T-cell receptor variable region may be derived from helper T-cell (CD4 positive T-cell), killer T-cell (CD8 positive T-cell), regulatory T-cell (Treg), Th17 cell, NKT cell, effector T-cell or γδT cell. The T-cell receptor derived from any of these T-cells can recognize an MHC molecule and bind it, and thus, they can be used as a constituent molecule of the T-cell receptor chimeric protein of the present invention.

In the present invention, the "Immunoglobulin Fc region" refers to an immunoglobulin Fc fragment, more specifically refers to, each of CH2 and CH3 constant domains of a natural immunoglobulin. As the immunoglobulin Fc region, human-derived one is preferably used and an immunoglobulin of a non-human animal, such as mouse immunoglobulin, can be used. The immunoglobulin is preferably IgG. Subclasses of human IgG include IgG1, IgG2, IgG3 and IgG4. Mouse immunoglobulin includes IgG1, IgG2a, IgG2b and IgG3. As human IgG, IgG1 and IgG3 are preferable since Fc regions of IgG1 and IgG3 strongly bind to an Fc receptor. Of them, IgG1 is preferable. As mouse IgG, IgG2a is preferable since the Fc region of IgG2a easily binds to an Fc receptor (FcR) of NK cell. Examples of the immunoglobulin Fc region include natural mutants, artificial mutants and truncated-form mutants; more specifically, include an Fc region having an amino acid sequence, which has an identity of 90% or more, 95% or more, 97% or more or 99% or more with that of a naturally-occurring immunoglobulin Fc region, as calculated by use of, e.g., BLAST (Basic Local Alignment Search Tool at the National Center for Biological Information)(using, for example, default, i.e., parameters of initial setting).

In the T-cell receptor chimeric protein, a linker may be present between T-cell receptor variable regions, between Fc regions of immunoglobulins, or between α chain and β chain if the T-cell receptor variable region has both α chain and β chain. It is sufficient if two proteins tandemly bind to each other via a linker. The linker is a peptide linker composed of a predetermined-length amino acid sequence. Although the number of amino acids is not limited, the number is 1 to 30, preferably 3 to 25, and further preferably 5 to 20. Although the types of amino acids are not limited, an amino acid having a short side chain and less reactivity, and an amino acid forming an α helix structure when connected, are preferable. Examples of these amino acids include glycine (G), serine (S), alanine (A), threonine (T), aspartic acid (D), lysine (K), glutamic acid (E), leucine (L) and methionine (M).

### (2) Method for specifying T-cell receptor (TCR)

In specifying a T-cell receptor in an autoimmune disease, a T-cell receptor variable region that may be used is a variable region of a T-cell receptor that can recognize a self-antigen expressed in a specific autoimmune disease. As such a T-cell receptor variable region, for example, a variable region of a T-cell receptor derived from a patient having a specific autoimmune disease, can be used. In the present invention, examples of the autoimmune disease to be treated include autoimmune diabetes, rheumatoid arthritis, multiple sclerosis, celiac disease, an inflammatory bowel disease such as Crohn's disease and ulcerative colon, psoriasis, type 1 diabetes mellitus, systemic lupus erythematosus, Hashimoto thyroiditis, myasthenia gravis, Guillain-Barre syndrome, autoimmune uveitis, primary biliary cirrhosis, autoimmune hepatitis, autoimmune hemolytic anemia, pernicious anemia, autoimmune thrombocytopenia, Graves' disease, autoimmune ovaritis, autoimmune orchitis, temporary arthritis, antiphospholipid syndrome, Wegner granulomatosis, Behcet's disease, scleroderma, polymyositis, dermatomyositis, rigid spondylitis, Sjogren's syndrome, herpes dermatitis, pemphigus vulgaris, vitiligo, psoriatic arthritis, osteoarthritis, steroid-resistant asthma, chronic obstructive pulmonary disease, alopecia areata and atherosclerosis.

In order to apply an appropriate treatment to each autoimmune-disease patient, lymphocytes are collected from the autoimmune-disease patient; the repertoires of T-cell receptors of the lymphocytes are identified; and the T-cell receptor variable region highly frequently found in the autoimmune-disease patient, may be used. In this case, the T-cell receptor recognizes a specific epitope of the self-antigen of the autoimmune-disease patient. DNA encoding such a T-cell receptor specific to a specific epitope can be obtained by exhaustively analyzing the T-cell receptor of the autoimmune-disease patient. Although the repertoires of T-cell receptors are 10¹⁸, they can be exhaustively analyzed at present. For example, T-cells are collected from a lymph node of an autoimmune-disease patient, and total mRNA is extracted and purified. Subsequently, cDNA is synthesized from the total mRNA by a reverse transcriptase to construct a cDNA library. In the cDNA library constructed, sequencing is carried out by a next-generation sequencer to analyze the repertoires of the T-cell receptors. The T-cell receptor highly frequently emerged in an autoimmune patient can be determined as the T-cell receptor highly specific to the autoimmune disease.

Subsequently, the full-length sequence of the T-cell receptor is cloned. At this time, the full-length sequence of the T-cell receptor is amplified by using a primer binding to the 5' end of DNA encoding a T-cell receptor variable region and a primer binding to the 3' end of a T-cell receptor constant region, and incorporated in a cloning vector. In this manner, a library of the full-length gene encoding the T-cell receptor is constructed. Genes of this full-length gene library are sequenced again. T-cell receptor highly frequently emerged in the T-cell receptor repertoire analysis is determined as a T-cell receptor specific to the autoimmune disease, and a clone having a sequence of gene of this T-cell receptor is selected as a clone for a T-cell receptor specific to the autoimmune disease.

If a donor cell is a target for an organ transplant rejection and T-cell receptor is specified, a T-cell receptor variable region capable of recognizing an antigen of a transplantation tissue derived from a donor may be used as the T-cell receptor variable region. As an example of the T-cell receptor variable region, a T-cell receptor variable region capable of recognizing a target cell derived from a donor can be used. In order to apply an appropriate treatment for organ transplant rejection to each patient, lymphocytes are collected from the organ-transplant recipient, the repertoires of T-cell receptors of the lymphocytes are identified, and a T-cell receptor variable region highly frequently found in the organ-transplant recipient may be used. In this case, the T-cell receptor recognizes a specific epitope of a transplantation tissue antigen derived from a donor. DNA encoding such a T-cell receptor specific to the specific epitope of a transplantation tissue antigen derived from a donor can be obtained by exhaustively analyzing the T-cell receptor of the organ-transplant recipient. Although the repertoires of T-cell receptors are 10¹⁸, they can be exhaustively analyzed at present. For example, T-cells are collected from a lymph node of an organ-transplant recipient and total mRNA is extracted and purified. Subsequently, cDNA is synthesized from the total mRNA by a reverse transcriptase to construct a cDNA library. For the cDNA library constructed, sequencing is carried out by a next-generation sequencer to analyze repertoires of the T-cell receptors. The T-cell receptor highly frequently emerged in an organ-transplant recipient can be determined as the T-cell receptor highly specific to the organ transplant rejection.

After a target cell derived from a donor and lymphocytes from an organ-transplant recipient are subjected to mixed culture, the lymphocytes are collected and the repertoires of T-cell receptors of the lymphocytes are identified. In this manner, T-cell receptor involved in an organ transplant rejection can be also identified. As a further simpler method, there is a method of subjecting the donor lymphocytes, which have been irradiated with X-rays in advance so as not to proliferate, together with the lymphocytes of an organ-transplant recipient to mixed culture (mixed lymphocyte reaction) and analyzing T cells reacted. In this manner, the T-cell receptor of the organ-transplant recipient involved in an organ transplant rejection can be identified.

Subsequently, the full-length sequence of the T-cell receptor is cloned. At this time, the full-length sequence of the T-cell receptor is amplified by using a primer binding to the 5' end of DNA encoding a T-cell receptor variable region and a primer binding to the 3' end of a T-cell receptor constant region, and incorporated in a cloning vector. In this manner, a library of full length genes encoding the T-cell receptor is constructed. Genes of this full-length gene library are sequenced again. A T-cell receptor highly frequently emerged in the T-cell receptor repertoire analysis is determined as a T-cell receptor specific to an infection, that is, a pathogen-specific T-cell receptor, and a clone having the sequence of gene of the T-cell receptor is selected as the clone for a clone for a pathogen-specific T-cell receptor. Note that organ transplant of "an organ transplant rejection" includes tissue transplant in the present invention.

In specifying the T-cell receptor of an allergic-disease patient, a T-cell receptor variable region capable of recognizing an allergen expressed in a predetermined allergic disease may be used as the T-cell receptor variable region. As the T-cell receptor variable region, a T-cell receptor variable region derived from a specific allergic-disease patient can be used. Examples of the allergic disease to be treated by the present invention include atopic dermatitis, allergic rhinitis, allergic conjunctivitis, allergic gastroenteritis, food allergy, bronchial asthma, childhood asthma, drug allergy, hives, metallic allergy and contact hypersensitivity.

In order to applying an appropriate treatment to each allergic-disease patient, lymphocytes are collected from an allergic-disease patient; the repertoires of T-cell receptors of the lymphocytes are identified; and the T-cell receptor variable region highly frequently found in the allergic-disease patient may be used. In this case, the T-cell receptor recognizes a specific epitope of an allergen of the allergic-disease patient. DNA encoding such a T-cell receptor specific to a specific epitope can be obtained by exhaustively analyzing the T-cell receptor of the allergic-disease patient. Although the repertoires of T-cell receptors are 10¹⁸, they can be exhaustively analyzed at present. For example, T-cells are collected from a lymph node of an allergic-disease patient and total mRNA is extracted and purified. Subsequently, cDNA is synthesized from the total mRNA by a reverse transcriptase to construct a cDNA library. In the cDNA library constructed, sequencing is carried out by a next-generation sequencer to analyze the repertoires of the T-cell receptors. A T-cell receptor highly frequently emerged in an allergic-disease patient can be determined as the T-cell receptor highly specific to the autoimmune disease.

Subsequently, the full-length sequence of the T-cell receptor is cloned. At this time, the full-length sequence of the T-cell receptor is amplified by using a primer binding to a 5' end of DNA encoding a T-cell receptor variable region and a primer binding to a 3' end of a T-cell receptor constant region and incorporated in a cloning vector. In this manner, a library of full-length gene encoding the T-cell receptor is constructed. Genes of this full-length gene library are sequenced again. A T-cell receptor highly frequently emerged in the T-cell receptor repertoire analysis is determined as a T-cell receptor specific to the allergic disease, and a clone having a sequence of gene of this T-cell receptor is selected as a clone for a T-cell receptor specific to the allergic disease.

Repertoire analysis of T-cell receptors can be carried out by use of, for example, IMGT/V-Quest tool (http://www.imgt.org/); and alternatively by a method described in International Publication No. WO2016/136716.

In the above method, after sequencing an autoimmune disease-specific T-cell receptor, an organ transplant donor-antigen specific T-cell receptor or an allergic disease-specific T-cell receptor is determined by the repertoire analysis of T-cell receptors, the full-length gene of the T-cell receptor is cloned again to construct a library. From the library, a clone having the sequence of a T-cell receptor involved in recognition of the autoimmune disease-specific antigen, organ transplant donor-antigen or allergen is selected.

Exhaustive analysis on the T-cell receptor of an autoimmune-disease patient, an organ-transplant recipient or an allergic-disease patient can be carried out in one to two weeks after blood is collected. Thereafter, the T-cell receptor may be cloned to produce a chimeric protein. A T-cell receptor chimeric protein, by which an individualized (custom-made) therapy suitable for a specific autoimmune-disease patient, organ-transplant recipient or allergic-disease patient is realized, can be obtained at earliest in about 3 to 5 weeks after blood collection. At this time, not only a T-cell receptor but also an immunoglobulin Fc region taken from a patient may be used. In either case, the T-cell receptor chimeric protein is meant to be derived from the patient. For the reason, side effects caused by immune reaction can be suppressed.

Further, the T-cell receptor can be specified by the following method. The appearance frequencies of TCRs in a specimen of an autoimmune-disease patient, organ-transplant recipient or allergic-disease patient are added up and averaged. The TCRs are aligned in the descending order of appearance frequency and, e.g., top 10 TCR are selected (total single analysis). The appearance frequencies of TCRs in a specimen of healthy-person peripheral blood are obtained and the TCRs are aligned in the descending order and e.g., top 10 are selected. From the TCRs selected in a patient tissue, TCRs overlapped with those selected from the healthy-person peripheral blood especially in the V region, are excluded and the remaining one(s) is/are determined as a specific TCR. This method can be applied to determination of not only T-cell receptor α chain variable region but also β chain variable region thereof.

More specifically, this is a method for specifying a T-cell receptor α chain variable region specific to an autoimmune-disease patient, organ-transplant recipient or allergic-disease patient, which comprises: identifying repertoires of a T-cell receptor α chain variable region of an autoimmune-disease patient, organ-transplant recipient or allergic-disease patient and repertories of T-cell receptor α chain of lymphocytes in the peripheral blood of a healthy person; and determining the T-cell receptor α chain variable region, which is present in lymphocytes of the autoimmune-disease patient, organ-transplant recipient or allergic-disease patient two times as large as that in the lymphocytes of the peripheral blood of the healthy person, as the T-cell receptor α chain variable region specific to the autoimmune-disease patient, organ-transplant recipient or allergic-disease patient. This is also a method for specifying a T-cell receptor α chain variable region specific to an autoimmune-disease patient, organ-transplant recipient or allergic-disease patient, by using a mixture of lymphocytes of a plurality of autoimmune-disease patients, organ-transplant recipients or allergic-disease patients and a mixture of lymphocytes of the peripheral blood sample of a plurality of healthy persons to determine the T-cell receptor α chain variable region, which is present in lymphocytes of the autoimmune-disease patient, organ-transplant recipient or allergic-disease patient two times as large as that in the lymphocytes of the peripheral blood of the healthy person, as the human common T-cell receptor α chain variable region specific to the autoimmune-disease patient, organ-transplant recipient or allergic-disease patient.

### (3) Production of T-cell receptor chimeric protein

If a plurality of patients are simultaneously subjected to exhaustive analysis of T-cell receptors, a large number of repertoires of T-cell receptors can be obtained and a large number of libraries of DNAs encoding T-cell receptor variable regions can be constructed. Thereafter, when T-cell receptor analysis of an autoimmune-disease patient, organ-transplant recipient or allergic-disease patient requiring a treatment is carried out; and the T-cell receptor analogous to the T-cell receptor of the patient or having an analogous sequence is found to be present in the library, the DNA is just employed to produce a T-cell receptor chimeric protein.

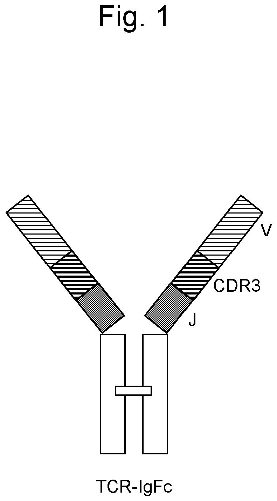

The T-cell receptor chimeric protein of the present invention comprises a monomer having a single fusion protein, which is formed by fusing an α chain variable region and/or β chain variable region of a T-cell receptor and an immunoglobulin Fc region, and if necessary, having a linker peptide, and a dimer having two fusion proteins. A dimer shown in Figure 1 is preferable. The T-cell receptor chimeric protein of the present invention can form a dimer by a disulfide bond. One of two T-cell receptor variable regions of a dimer may be α chain and the other may be β chain.

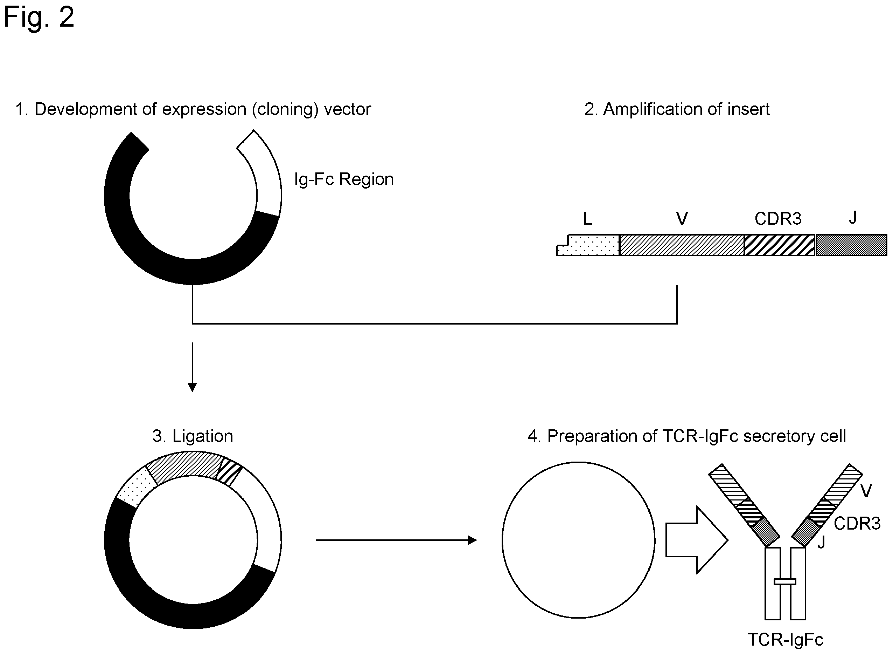

The T-cell receptor chimeric protein of the present invention can be produced by a method for producing a fusion protein known to artisans, for example, a chemical synthesis method, a method using a gene recombination technique, and preferably produced by gene recombination technique. The method using a gene recombination technique is shown in Figure 2. When the T-cell receptor chimeric protein of the present invention is produced by a gene recombination technique, DNA encoding a T-cell receptor α and/or β chain variable regions and an immunoglobulin Fc region, and if necessary, a linker peptide, are ligated inframe, DNA encoding the fusion protein is introduced into an expression vector to produce a recombinant vector, and the recombinant vector is further introduced into a host such as an animal cell, an insect cell, a plant cell, a yeast cell and a bacteria cell and expressed therein.

As the vector, any vector replicable in a host cell such as a plasmid, a phage and a virus can be used. The vector contains a promotor, a replication origin and a selective marker; and if necessary, may contain an enhancer, a transcription termination sequence (terminator), a ribosome binding site and polyadenylation signal.

A T-cell receptor chimeric protein produced can be, if necessary, isolated and purified by isolation and purification means known to those skilled in the art. Examples of isolation and purification methods include affinity chromatography, ion exchange chromatography, gel filtration chromatography, hydrophobic chromatography, mixed-mode chromatography, dialysis, fractionation by precipitation and electrophoresis. These means may be used appropriately in combination to isolate and purify the T-cell receptor chimeric protein of the present invention.

Further, the T-cell receptor chimeric protein of the present invention can be expressed in a cell-free translation system (cell-free system).

The T-cell receptor chimeric protein of the present invention may be chemically modified as far as the modification is known to those skilled in the art. Examples of chemical modification include polyethylene glycosylation (PEG), glycosylation, acetylation and amidation.

The present invention comprises a method for producing a T-cell receptor chimeric protein being a fusion protein of a T-cell receptor variable region and an immunoglobulin Fc region, the method comprising: cloning DNA encoding an autoimmune disease antigen-specific T-cell receptor or allergic disease-specific T-cell receptor, which recognizes an autoimmune disease-specific antigen or allergic disease-specific antigen, from T cells collected from an autoimmune-disease patient or an allergic-disease patient; ligating DNA encoding the immunoglobulin Fc region; introducing the ligated product into an expression vector; introducing the expression vector into a host cell; and allowing the expression vector in the host cell. The present invention also comprises a method for producing a T-cell receptor chimeric protein being a fusion protein of a T-cell receptor variable region and an immunoglobulin Fc region, the method comprising: analyzing the repertoire of a T cell-receptor (that an autoimmune-disease patient or an allergic-disease patient has) of T cells collected from an autoimmune-disease patient or an allergic-disease patient; cloning the T-cell receptor, which highly frequently emerges in the autoimmune-disease patient or allergic-disease patient, as a specific T-cell receptor having a high specificity to the autoimmune disease or allergic disease; ligating DNA encoding the immunoglobulin Fc region and introducing a ligated product into an expression vector; introducing the expression vector into a host cell; and allowing the vector to express in the host cell.

The present invention further comprises a method for producing a T-cell receptor chimeric protein being a fusion protein of a T-cell receptor variable region and an immunoglobulin Fc region, the method comprising: cloning DNA encoding a specific T-cell receptor capable of recognizing an organ transplant rejection specific antigen/MHC complex; ligating DNA encoding an immunoglobulin Fc region, introducing a ligated product into an expression vector; introducing the vector into a host cell; and allowing the vector to express in the host cell. The present invention also comprises a method for producing a T-cell receptor chimeric protein being a fusion protein of a T-cell receptor variable region and an immunoglobulin Fc region, the method comprising: analyzing the repertoire of a T-cell receptor of an organ-transplant recipient; cloning the T-cell receptor highly frequently emerged as a specific T-cell receptor having a high specificity to organ transplant rejection; ligating DNA encoding the immunoglobulin Fc region and introducing a ligated product into an expression vector, introducing the vector into a host cell; and allowing the vector to express in the host cell.

The present invention comprises a complex of a T-cell receptor chimeric protein capable of recognizing an autoimmune disease-specific antigen or allergic disease-specific antigen, and MHC of antigen-presenting cell. The present invention also comprises a method for producing a complex of T-cell receptor chimeric protein and an antigen presenting cell, by bringing a T-cell receptor chimeric protein capable of recognizing an autoimmune disease-specific antigen or allergic disease-specific antigen, *in-vitro,* into contact with an antigen presenting cell.

The present invention further comprises a complex of T-cell receptor chimeric protein capable of recognizing an antigen specific to an organ transplant rejection and an antigen presenting cell. The present invention also comprises a method for preparing a complex of T-cell receptor chimeric protein and an antigen-presenting cell by bringing an antigen specific to an organ transplant rejection and a T-cell receptor chimeric protein capable of recognizing an MHC complex, *in-vitro.*

### 2. Use of T-cell receptor chimeric protein

### (1) Incorporation of T-cell receptor chimeric protein into cell and down modulation of MHC complex on cell surface

Expression of an MHC complex on the cell surface can be down-modulated by a T-cell receptor chimeric protein and reduced. In addition, a T-cell receptor chimeric protein bound to an MHC complex can be introduced into a cell.

The present invention comprises a down-modulating agent for an MHC complex in a cell, which contains a T-cell receptor chimeric protein as an active ingredient. The T-cell receptor chimeric protein of the present invention reduces the expression of a complex of antigen and an MHC molecule or a complex of an antigen presenting antigen peptide or antigen peptide and an MHC molecule on an antigen-presenting cell or a disease target cell recognized by a pathogenic T cell, thereby regulating reactivity of a target pathogenic T cell, which recognizes the MHC complex in an antigen or antigen-peptide specific manner, with the result that the function of pathogenic T cell is reduced. Herein, the "expression of a complex of an MHC molecule is reduced in an antigen or antigen-peptide specific manner" refers to specifically reducing expression only of a complex of a predetermined antigen and an MHC molecule. The present invention comprises an MHC molecular complex down-modulating agent containing a T-cell receptor chimeric protein as an active ingredient and an agent for decreasing pathogenic T-cell function, containing a T-cell receptor chimeric protein as active ingredient. The MHC molecular complex down-modulating agent is also referred to as an MHC molecular complex regulator. The regulation is also referred to as modulation. Herein, the pathogenic T cell refers to a T cell involved in immune reactions of an autoimmune disease and an allergic disease and causing a disease or an organ transplant rejection in organ transplant. The T-cell receptor chimeric protein of the present invention may inhibit function of the T cell in an antigen peptide specific manner and in competition with a pathogenic T cell, decrease the function. The MHC molecule includes not only an MHC class I molecule and an MHC class II molecule but also a non-classical MHC molecule. An MHC class II molecule is mostly involved in an autoimmune disease and an allergic disease; whereas, an MHC class I molecule is mostly involved in an organ transplant rejection.

The T-cell receptor chimeric protein to be used as an active ingredient of a down-regulating agent for the MHC complex is preferably a multimer such as a dimer or a tetramer. A multimer is preferable because it has a satisfactory clustering efficiency and is highly effective in reducing expression of an MHC complex and T cell function.

A T-cell receptor binds to an MHC complex and transmits a signal to a T-cell to produce its function. The T-cell receptor chimeric protein of the present invention binds to an MHC complex on the surface of a cell, and then, is incorporated in the cell to reduce the expression of the MHC complex on the cell surface. The T-cell receptor chimeric protein bound to the MHC complex on the cell surface is incorporated in the cell, 1 to 10 hours later, preferably 4 to 8 hours later and further preferably 6 hours later.

The MHC complex, to which the T-cell receptor chimeric protein of the present invention is bound, is not limited to a class I molecule and may be class II molecule. H-2K, D, L, I-A, I-E may be used in mice; whereas, HLA-A, B, C, DR, DQ, DM and E may be used in humans. Alternatively, a non-classical MHC molecule may be acceptable. As the non-classical MHC molecule, a class Ib molecule is mentioned, which includes CD1, MR1, H2-M3, HLA-G (Biochemistry vol. 81, No. 3, pp.189-199, 2009). The target cell is not limited to an autoimmune disease, organ transplant rejection and allergic disease. All types of cells including normal cells are targeted.

The present invention comprises a method for reducing expression of an MHC of a cell, which is a target of an antigen-presenting cell or pathogenic T cell, by use of a T-cell receptor chimeric protein, thereby decreasing function of the T cell, and comprises a pharmaceutical composition containing a T-cell receptor chimeric protein as an active ingredient and possibly used as an agent for decreasing function of a T cell. The pharmaceutical composition can be used for treatment of an autoimmune disease, treatment or suppression of an organ transplant rejection or treatment of an allergic disease or suppression of allergy. More specifically, the present invention comprises an autoimmune disease therapeutic agent, a therapeutic agent or suppressant for an organ transplant rejection or a therapeutic agent for an allergic disease or an allergy suppressant.

A dosage form of the pharmaceutical composition of the present invention is not limited, and various dosage forms may be used depending on the usage. Examples of oral preparations include tablets, powders, granules, fine granules and capsules. Examples of parenteral preparations include injections, inhalation powders, inhalation liquids, eye drops, solutions, lotions, sprays, nasal drops, infusions, ointments, suppositories and plasters. The pharmaceutical composition of the present invention may be prepared by a method known in the field of pharmaceutical science in accordance with the dosage form. Examples of a pharmaceutical additive include excipients, disintegrants, binders, lubricants, diluents, buffers, tonicity agents, preservatives, stabilizers and solubilizing agents. As the pharmaceutical additive, physiological saline and injection solvents are included.

The pharmaceutical composition of the present invention may be administered by various methods depending on the usage. Examples of the administration method include oral administration, intravenous administration, subcutaneous administration, intramuscular administration, intraperitoneal administration and topical administration.

When the pharmaceutical composition of the present invention is used in treatment for an autoimmune disease, an organ transplant rejection or allergic disease, the dose of protein of the present invention serving as an active ingredient is appropriately determined in accordance with, e.g., the age, sex and body weight of the patient, the severity of the disease, the dosage form and administration route. For example, when the composition is orally administered to an adult, the dose may be determined within the range of 0.1 µg/kg to 1000 mg/kg/day. Daily dose may be divided into one, two or three portions and administered. When the composition is parenterally administered to an adult, the dose may be determined within the range of 0.01 µg/kg to 1000 mg/kg/day. Daily dose for parenteral administration may be determined, depending on the dosage form, in the range of preferably 0.1 µg/kg to 10 µg/kg/day, 1 µg/kg to 100 µg/kg/day, or 10 µg/kg to 1000 µg/kg/day.

The present invention comprises a method for reducing expression, comprising: binding a T-cell receptor chimeric protein being a fusion protein of a T-cell receptor variable region and an immunoglobulin Fc region as an active ingredient, to an MHC complex of a target cell to induce down modulation of the MHC complex. The present invention also comprises a method of allowing a T-cell receptor chimeric protein to bind to an MHC complex on a target cell and incorporate in the target cell.

### (2) Detection of self-antigen causing an autoimmune disease, a donor target cell of an organ transplant rejection or allergen

Since the T-cell receptor chimeric protein of the present invention binds to a complex of an MHC molecule of a target cell, which serves as a target for pathogenic T cell of an autoimmune disease, and a self-antigen, a complex of an MHC molecule of a target cell derived from a donor, which causes an organ transplant rejection, and a donor-specific antigen, or a complex of an MHC molecule of a target cell, which serves as a target for pathogenic T cell of an allergic disease, and an allergen, the T-cell receptor chimeric protein can be used for detecting these target cells in a test subject. If a target cell serving as a target for pathogenic T cell of an autoimmune disease is detected, it can be determined that the test subject has an autoimmune disease. If a donor-derived target cell causing an organ transplant rejection is detected, it can be determined that a rejection (a cause of organ transplant rejection) occurs in the test subject. If a target cell serving as a target for pathogenic T cell of an allergic disease is detected, it can be determined that the test subject has an allergic disease. The determination that a test subject has an autoimmune disease is referred to as detection of the autoimmune disease. The determination that a rejection (a cause of organ transplant rejection) occurs in the test subject is referred to as detection of a rejection. The determination that the test subject has an allergic disease is referred to as detection of the allergic disease. A self-antigen causing an autoimmune disease, a donor specific antigen causing an organ transplant rejection and allergen causing an allergic disease can be detected.

In the case of using the T-cell receptor chimeric protein to detect a target cell of an autoimmune disease, a donor-derived target cell causing an organ transplant rejection or a target cell of an allergic disease, the T-cell receptor chimeric protein may be labeled with a fluorescent dye, a quenching dye, a fluorescent protein, an enzyme such as alkaline phosphatase (ALP) and horseradish peroxidase (HRP) and then put in use. Labeling with a marker substance can be carried out by a method for labelling a protein known to artisans and may be carried out by the biotin-avidin (streptavidin) system.

Detection may be made by use of FACS or a flow cytometer, or by an immunocytochemical method. As the flow cytometer, for example, FACSCant II (manufacture by Becton, Dickinson and Company) may be used. In the measurement by an immunocytochemical method, a target cell of an autoimmune disease, a donor-derived target cell causing an organ transplant rejection or a target cell of an allergic disease is fixed onto a glass slide and measured. In the immunocytochemistry, staining can be determined by a microscope or the naked eyes or may be determined by an appropriate optical measurement device.

If cells were collected from a biological sample such as blood or a tissue of a test subject and brought into contact with a T-cell receptor chimeric protein and a cell binding to the T-cell receptor chimeric protein was found to be present, it is determined that the cell is a target cell causing an autoimmune disease, and that a self-antigen causing an autoimmune disease is expressed. If a donor-derived target cell is detected in a biological sample of a test subject, it is determined that an organ transplant rejection occurs in the test subject. If cells were collected from a biological sample such as blood or a tissue of a test subject and brought into contact with a T-cell receptor chimeric protein and a cell binding to the T-cell receptor chimeric protein was found to be present, it is determined that the cell is a target cell causing an allergic disease, and that an allergen causing an allergic disease is expressed. Assistance data for diagnosing an autoimmune disease, an organ transplant rejection or allergic disease can be obtained by detection using the T-cell receptor chimeric protein.

The present invention comprises a reagent for detecting an autoimmune disease, organ transplant rejection or allergic disease containing a T-cell receptor chimeric protein, and preferably, a labeled T-cell receptor chimeric protein.

The patient determined to have an autoimmune disease or an allergic disease may be treated by the agent for decreasing T-cell function of the present invention and can be treated in combination with a symptomatic treatment such as anti-inflammatory therapy. The patient determined to have an organ transplant rejection may be treated by the T cell function down-regulating agent of the present invention and can be treated by administering an immunosuppressant.

### Examples

The present invention will be more specifically described by way of the following examples; however, the present invention is not limited by these examples.

### [Example 1]

### 1. T-cell receptor (TCR) repertoire analysis

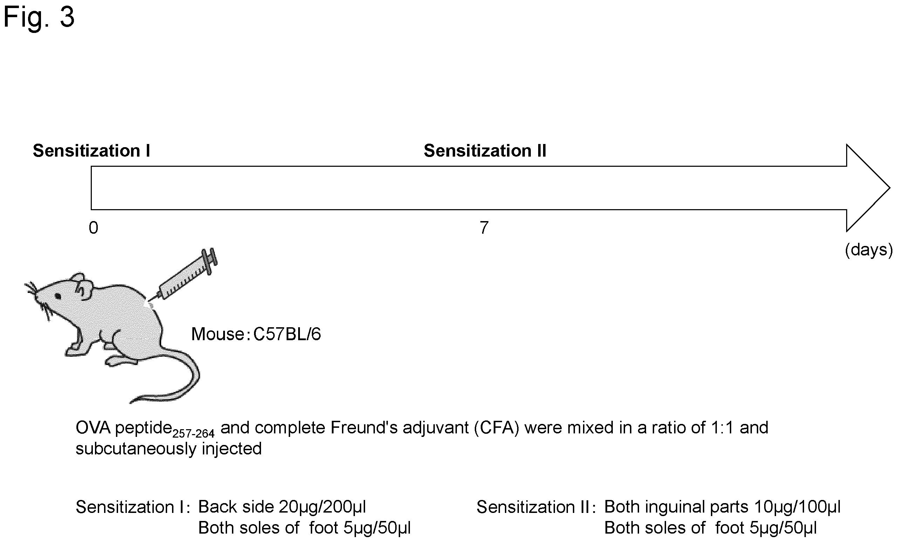

With OVA peptide (257-264: SIINFEKL), an equivalent amount of e.g., complete Freund's adjuvant (CFA) was blended to prepare an emulsion. C57BL/6 mice were subcutaneously vaccinated (sensitized) with the emulsion on Day 0 and Day 7. The vaccination (sensitization) schedule is shown in Figure 3. On Day 15, lymphocytes were collected from regional lymph nodes and the spleen. Two mice were used per group. As a control, lymphocytes were collected from the spleen of mouse OT-1, to which the OVA peptide (257-264)-specific TCR gene was introduced.

Total RNA was extracted from each of the above samples by a routine method and a cDNA library was constructed. An adapter was provided in accordance with "gene specific unbiased amplification method" and PCR was carried out to amplify the gene of a TCR chain. This was used as an analysis sample.

In the gene specific unbiased amplification, an adapter is attached to the cDNA library, and then, the antisense strand (or sense strand) of the double-stranded adapter is digested with an enzyme. Using a primer prepared based on the nucleotide sequence of the remaining adapter and a TCR-specific primer, the adapter ligation PCR method is performed. In this manner, gene-specific amplification can be made while suppressing binding of a nonspecific primer. The gene specific unbiased amplification method is described in International Publication No. WO2016/136716.

Specifically, gene specific unbiased amplification is performed in accordance with the following method:
(i) a step of ligating any one of the double-stranded adapter DNAs of the following [1] to [3] to the both ends of a double-stranded cDNA,
(ii) a step of treating the gene prepared by ligating the double-stranded adapter DNA is treated with uracil DNA glycosylase (UNG) and further applying a heat treatment to decompose the antisense strand of the adapter DNA, and
(iii) a step of performing PCR amplification using a forward primer constituted of part or whole of the sequence of the antisense strand of the double-stranded adapter DNA and a reverse primer specifically annealing to the target gene.

In this method, an extension reaction with the forward primer alone does not occur and an extension reaction by the reverse primer alone occurs. After the complementary strand of the sense strand of the adapter is formed, the forward primer anneals to the complementary strand. In this manner, an extension reaction takes place. Since extension by the reverse primer and extension by the forward primer occur in this order in a single direction, the target gene can be amplified by the PCR method in a single direction without applying a bias.
[1] Double-stranded adapter DNA to be used for unbiased gene amplification having the following characteristics;
   (a) a sense strand and an antisense strand are annealed and have the same base length or the sense strand is longer,
   (b) the base length of the sense strand is 15 to 40 bp,
   (c) the antisense strand contains a plurality of uracil bases, which are removed by treating the adapter with uracil DNA glycosylase (UNG). Thereafter heat treatment is applied to decompose the antisense strand,
   (d) at least one of the ends of the adapter DNA is a blunt end,
   (e) the adapter DNA binds to a target gene to be amplified at the other end, and
   (f) part or whole of the sense strand is the forward primer sequence to be used for gene amplification.
[2] The double-stranded adapter DNA according to [1], wherein the number of uracil bases contained in the antisense strand occupies 10 to 25% of the number of the bases of the antisense strand and an uracil base is present in every 5 to 10 bases.
[3] The double-stranded adapter DNA according to [1] or [2], wherein a phosphate group binds to the 5' end of the antisense strand, and an amino group binds to the 3' end.

Samples were analyzed by a next generation sequencer, i.e., illumine MiSeq, in accordance with the manufacturer's protocol.

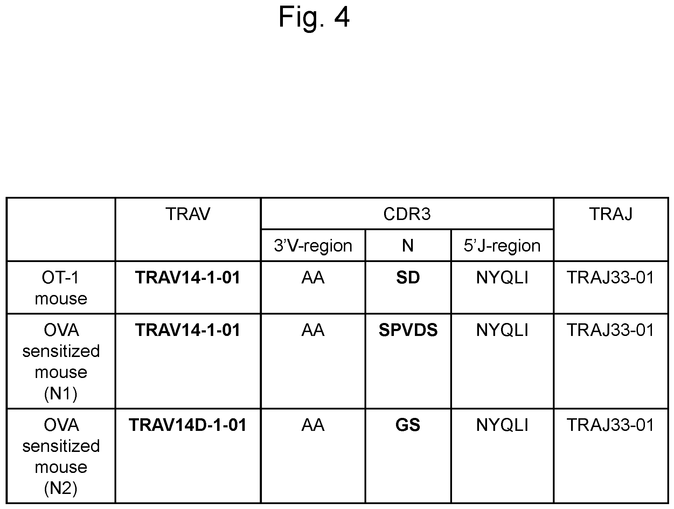

T-cell receptors (TCR) analyzed are shown in Figure 4.

The T-cell receptors to OVA peptides (SIINFEKL) (SEQ ID NO: 1) of both sensitized mice and OT-1 mouse were found to be analogous in V region and J region of α chain but different in CDR3 region. TCR of OT-1 mouse was as follows:
V region: TRAV14-1-01, J region: TRAJ33-01 CDR3 (amino acid sequence): AASDNYQLI (SEQ ID NO: 2)
TCR of mouse N1 sensitized with OVA peptide (SIINFEKL) (SEQ ID NO: 1) was as follows:
V region: TRAV14-1-01, J region: TRAJ33-01 CDR3 (amino acid sequence): AASPVDSNYQLI (SEQ ID NO: 3)
TCR of mouse N2 sensitized with OVA peptide (SIINFEKL) (SEQ ID NO: 1) was as follows:
V region: TRAV14D-1-01, J region: TRAJ33-01 CDR3 (amino acid sequence): AAGSNYQLI (SEQ ID NO: 4).
Then, the TCR of mouse N1 sensitized with OVA peptide (257-264) was arbitrarily selected and subjected to the following experiments.

### 2. Preparation of T-cell receptor chimeric protein

### 1) Gene cloning of T-cell receptor and construction of expression plasmid

A TCR having V region: TRAV14-1-01, J region: TRAJ33-01, CDR3 (amino acid sequence): AASPVDSNYQLI (SEQ ID NO: 3) was used. The region including L region to part of C region of TCRα chain was cloned from cDNA library of a spleen sample on Day 15 after transplantation with EL4 by using the following PCR primers.

### Primer sequences:

TO1771 (V region sense strand, EcoRI site was attached): GAG AAT TCG CAG CAG CAG GTG AGA CAA AG (SEQ ID NO: 5),

TO1881 (antisense strand including part of C region, Bgl II site was attached): GAT AGA TCT TTC TGG GTT CTG GAT GTC TGG CTT TAT AAT TAG (SEQ ID NO: 6).

A T-cell receptor chimeric protein plasmid (designated as TP352) was constructed by inserting cDNA into an EcoRI-BglII site of a commercially available pFUSE-mIgG2A-Fc plasmid (company: Invivogen).

The method for preparing a T-cell receptor chimeric protein is schematically shown in Figure 2.

### 2) Production and purification of T-cell receptor chimeric protein (TCR-IgFc fusion protein)

The T-cell receptor chimeric protein plasmid was introduced into HEK293 cells, which were then cultured in a medium containing ultralow IgG-FCS (added in order to avoid contamination with bovine IgG in the following purification step) for 4 days. The culture supernatant was recovered and purified by a Hi Trap protein G column (company: GE Healthcare, Japan) in accordance with the manufacturer's protocol.

The T-cell receptor chimeric protein is a chimeric protein obtained by binding TCR V region, CDR3, J region and part of the C region to IgFc moiety.

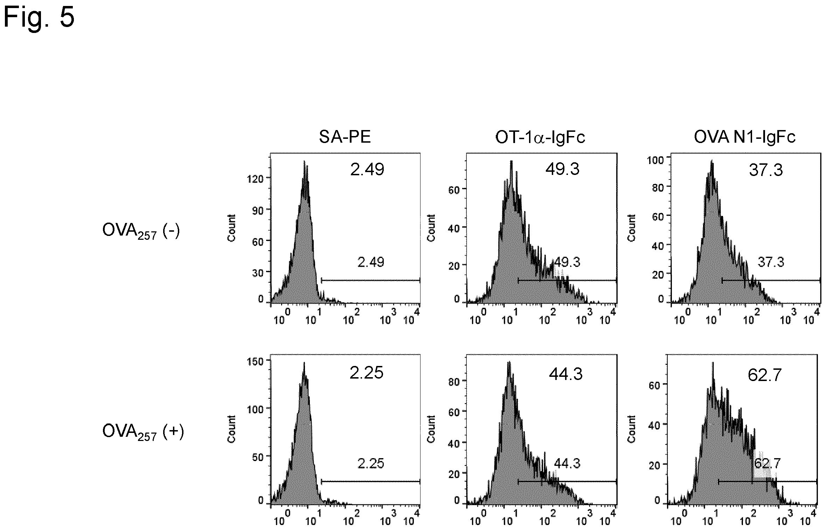

### 3. Binding of OVA peptide (SIINFEKL)-specific TCR-IgF (Figure 5)

Mouse thymoma cells EL4 are tumor cells expressing H-2K^{b}. To EL4 cells, 50 µg/mL OVA peptide (SIINFEKL) was added. The cells were cultured at 37°C for one hour. Thereafter, TRAV14-1-01-CDR3 (AASPVDSNYQLI)-TRAJ33-01 identified from OVA sensitized mouse N1 and mIgG2a Fc chimeric protein (TRAV14 N1-IgFc) or TCR α chain derived from OT-1 mouse and mIgG2a Fc chimeric protein (TRAV14-1-01-CDR3 (AASDNYQLI)-TRAJ33-01-IgFc (TRAV14-Fc) were biotinylated and then added in an amount of 2.5 µg/mL. Thereafter, the mixture was allowed to stand still at 4°C for one hour. The cells were washed, labeled by adding streptavidin-PE, allowed to stand still at 4°C for 20 minutes and detected by a flow cytometer.

In OT-1α-IgFc, no difference was observed between the presence or absence of OVA peptide; whereas, in TRAV14 N1-Fc, the binding property of OVA N1-IgFc (TRAV14 N1-IgFc) increased from 37.3% to 62.7% in the presence of OVA peptide. From the results, it was demonstrated that TRAV14 N1-IgFc binds to an MHC class I complex in an antigen-peptide specific manner. The numerical values in the figure represent the ratio (%) of cells to which TCR-IgFc is bound.

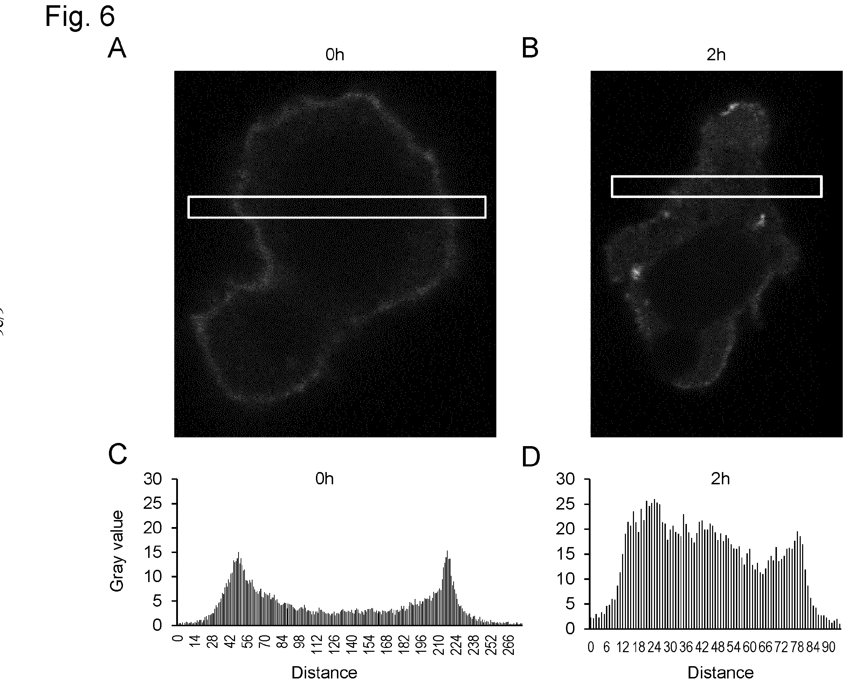

### 4. Induction of migration of MHC class I into cells by TCR-IgFc in an antigen peptide dependent manner (Figure 6)

To mouse thymoma cells EL4, 50 µg/mL OVA peptide (SIINFEKL) was added. The cells were cultured at 37°C for one hour. Thereafter, 2.5 µg/mL OVA peptide-specific TCR chimeric protein TRAV14 N1-Fc (TRAV14-1-01-CDR3 (AASPVDSNYQLI)-TRAJ33-01-IgFc) was added and the cells were cultured at 37°C for further two hours. The cells were washed with 2% FCS/PBS, and thereafter, 100 µl of Fix/Perm buffer (company: BD) was added. The cells were allowed to stand still at 4°C for 30 minutes. In this manner, fixation and permeabilization of the cells were carried out. Thereafter, labelling with biotinylated anti-H-2K^{b}D^{b} antibody, and further with streptavidin-Dyelight 488 was carried out. Observation was carried out by TCP SP8 (Leica) (Figures 6A, B) and image analysis was carried out by Image J Plot Profile (Figures 6C, D). It was found that localization of MHC class I within a cell is induced by addition of OVA N1-IgFc.

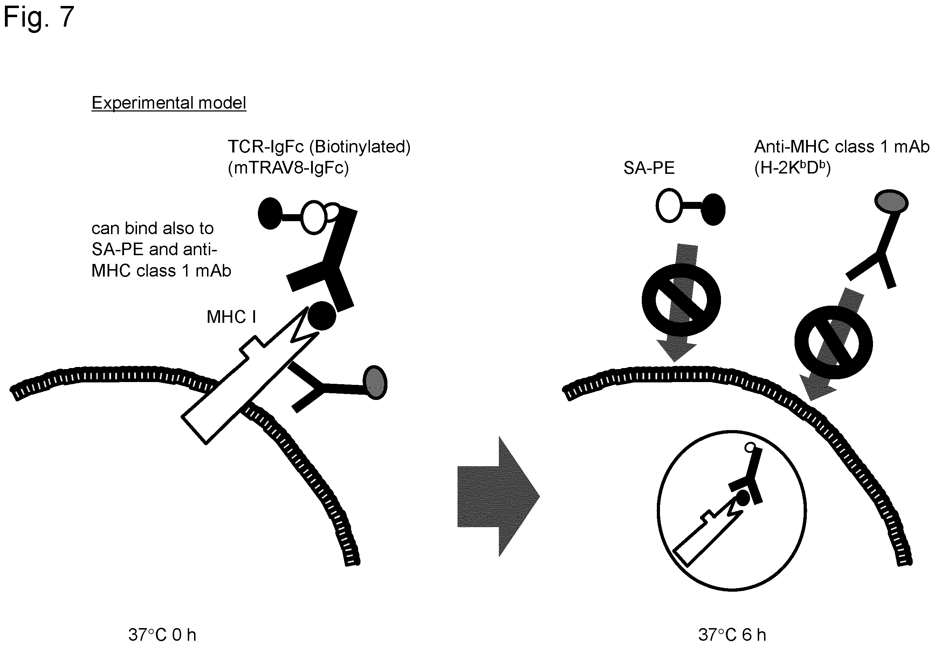

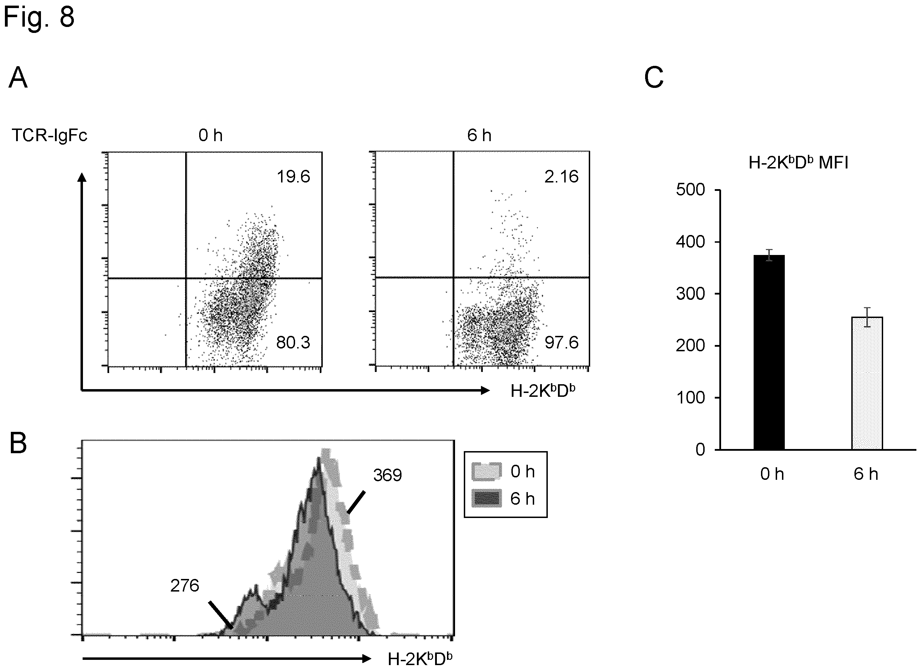

### 5. Behavior of TCR chimeric protein complex bound to MHC molecule (Figure 7, Figure 8)

Mouse thymoma cells, EL4, are collected and 2 × 10⁵ cells are suspended in 5% FCS/RPMI. TCR chimeric protein TCR-IgFc (mTRAV8-Fc), which was found to bind to MHC class I of EL4 cells, was biotinylated; and 2.5µg/mL biotinylated TCR-IgFc (bio-mTRAV8Ig) was added in the cell suspension and allowed to stand still at 37°C for 6 hours. The cells were washed twice with 2% FCS/PBS, streptavidin (SA)-Phycoerythrin (PE) and FITC-anti-MHC class I (H-2K^{b}D^{b}) antibody were added each in an amount of 2.5 µg/mL and allowed to stand still at 4°C for 20 minutes to stain the cells. After the cells were washed three times, the binding properties of TCR-Fc (mTRAV8-Fc) and MHC class I antibody were evaluated by a flow cytometer. The control group was prepared by adding biotinylated TCR-IgFc (io-mTRAV8 Fc)), allowing it to stand still at 4°C for one hour and thereafter, adding SA-PE and FITC-anti-MHC class I (H-2K^{b}D^{b}) antibody. Figure 7 shows the process for the experiment.

As shown in Figure 8A, at the time point of 0 h, TCR-IgFc (mTRAV8-Fc), which is bound to MHC class I, is present on a cell surface (19.6%). In contrast, at the time of 6 h, it is presumed that an MHC complex, to which mTRAV8-Fc is bound, migrated into cells. Thus, the complex was virtually not detected on cell surface (2.16%). At the time point of 0 h, MHC class I was extremely highly expressed (Figure 8B, histogram, dashed line). In contrast, at the time point of 6 h, the expression of MHC class I on a cell surface decreases by the migration of MHC class I, to which mTRAV8-Fc is bound, into the cells. More precisely, it was confirmed that expression is regulated. The numerical values in Figure 8A represent the ratios (%) of positive cells; whereas, the numerical values in Figure 8B represent mean fluorescence intensities (MFI). Figure 8C is a graph showing MFI of MHC class I, which is migrated within a cell and expressed on a cell surface. From these results, the expression (level) of MHC class I expressed on cell surface decreases by binding of TCR-Fc to MHC class I.

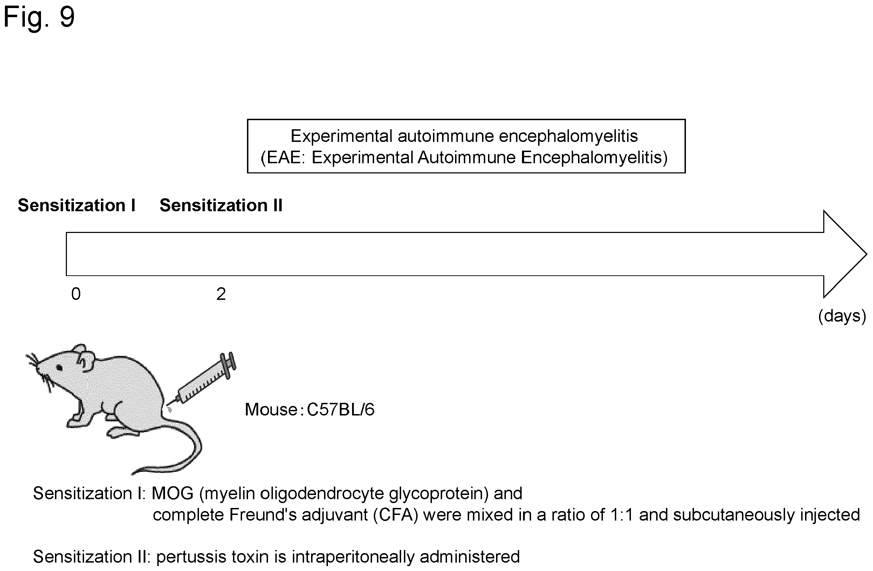

### 6. Preparation of experimental autoimmune encephalomyelitis (EAE) model (Figure 9)

To 6-8 week-old female C57BL/6 mice, 50 µg of myelin oligodendrocyte glycoprotein (MOG) 35-55 peptide (MOG: MEVGWYRSPFSRVVHLYRNGK) (SEQ ID NO: 7) (company: ANASPEC), which was suspended in complete Freund's adjuvant (CFA) containing dead bacterial cells (5 mg/mL) of *mycobacterium tuberculosis* (H37Ra), was subcutaneously administered. On the same day and Day 2 after administration, 400 ng of pertussis toxin (BD Difco) was intraperitoneally administered to sensitize the mice (Stromenes IM et al., Nat protoc; 1: 1810-9, 2006). Onset of EAE was evaluated based on the clinical score indicating the degree of paralysis (0: asymptomatic, 1: loss of tension in tail, 2: paralysis of single lower limb, 3: paralysis of both lower limbs, 4: cannot walk by itself, 5: lethal). The process for the experiment is shown in Figure 9.

### 7. Preparation of chimeric protein based on T-cell receptor accelerated in EAE

T-cell receptor chimeric protein to α chain (TRAV9-2D-2 + CDR3 (VYFCALRSYNFG (SEQ ID NO: 10)) (Vα3.2Jα18) and β chain TRBV16-04 + CDR3 (CASSLDCGANP (SEQ ID NO: 11)) + TRBJ1-1-01) (Vβ11DJβ1.1) (Battelli E et al., J Exp Med 197: 1073-1081, 2003) of a T cell-receptor (TCR), which were reported to be accelerated in EAE, were prepared. To pFUSE-mIgG2A-Fc plasmid (company: Invivogen), the sequence of TCR α chain (Vα3.2Jα18: hereinafter, TRAV9D-2) or TCR β chain (Vβ11DJβ1.1: hereinafter, TRBV16-04) was ligated to prepare Vα3.2Jα18-IgFc plasmid (TP359) and β11DJβ1.1-IgFc plasmid (TP360). HEK293 cells were transfected with the plasmid DNAs. The following day, the medium was exchanged with DMEM containing Ultralow IgG and the cells were cultured for 5 days. The culture supernatant was recovered and protein purification was carried out by affinity chromatography using Protein G sepharose (GE Healthcare). The presence of a fraction of T-cell receptor chimeric protein was confirmed by electrophoresis according to SDS-PAGE.

T-cell receptor chimeric protein (TRAV9D-2-Fc fusion protein, TRBV16-04-Fc fusion protein), which was prepared based on a T-cell receptor, was biotinylated by use of a commercially available biotinylation kit (Dojindo biotin labeling kit; Wako Pure Chemical Industries Ltd.).

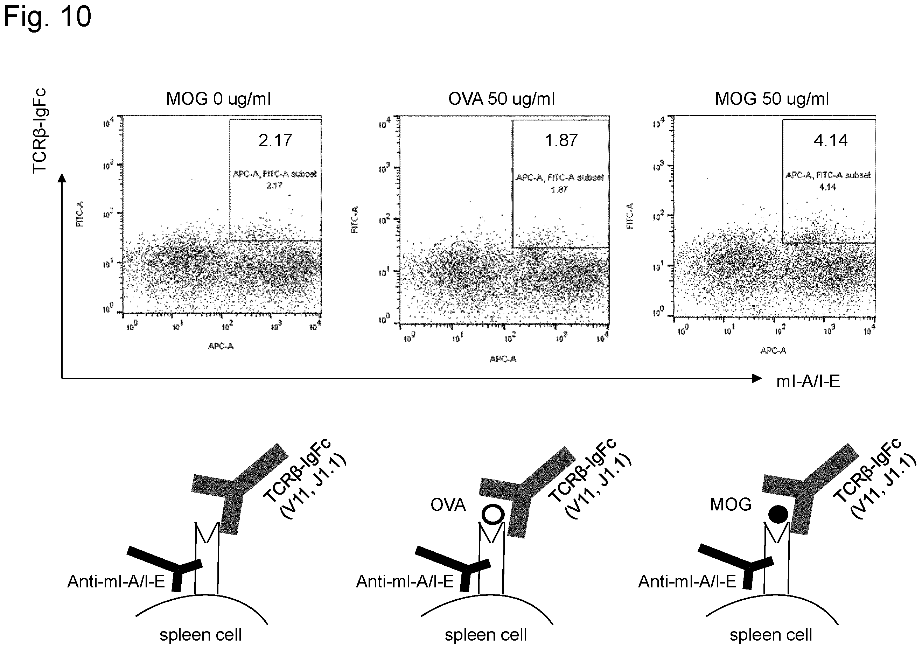

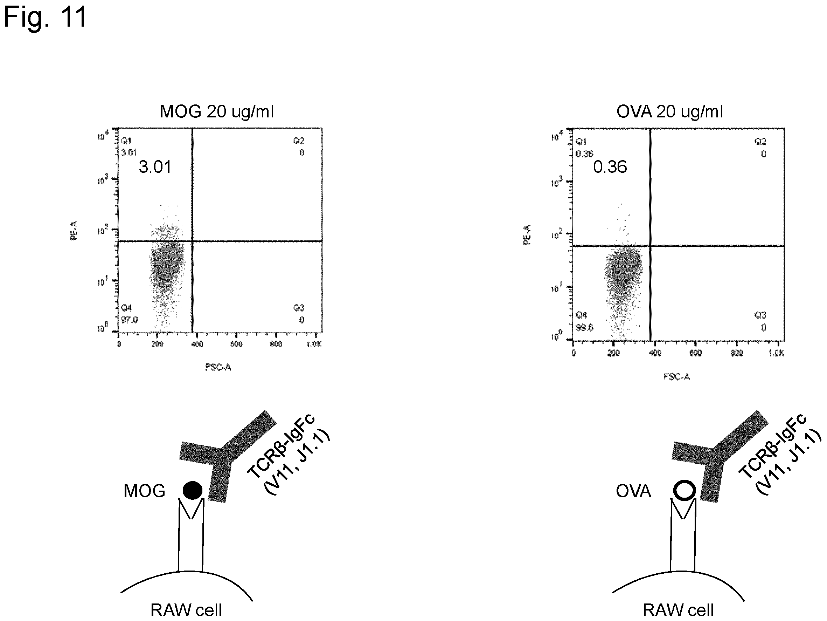

### 8. Binding of antigen peptide-specific T-cell receptor chimeric protein (Figure 10, Figure 11)

In order to check whether or not TCR β-IgFc chimeric protein (TRBV16-04-Fc chimeric protein) has specificity to MOG peptide, spleen cells were collected from C57BL/6 mice. To spleen cells (3 × 10⁵ cells), 20 µg/mL MOG peptide or OVA peptide was added. The cells were cultured at 4°C for one hour and washed with PBS containing 2% FBS. Thereafter, in order to inhibit an Fc receptor, the cells were pretreated with 2.4G2 antibody used as an Fc block, and 2.5 µg/mL TCR β-IgFc chimeric protein (TRBV16-04-Fc chimeric protein) was added. The cells were cultured at 4°C for one hour and washed with PBS containing 2% FBS. Labelling was carried out by adding Dye Light, Alexa 488 (BioLegend, Clone M5/114.15.2). Analysis was made by use of FACS CantoII (BD Biosciences)and FlowJo software (TreeStar).

As shown in Figure 10, it was confirmed that binding of TRBV16-04-Fc chimeric protein increases by addition of MOG peptide. The numerical values represent the ratio (%) of positive cells.

The same experiment was carried out using a cultured cell line, i.e., RAW264.7 cells. To RAW264.7 cells (3 × 10⁵ cells), 20µg/mL MOG peptide or OVA peptide was added. The cells were cultured at 4°C for one hour, and then, washed with PBS containing 2% FBS. Thereafter, in order to inhibit an Fc receptor, the cells were pretreated with 2.4G2 antibody used as a Fc block, and 2.5µg/mL TRBV16-04-Fc was added. The cells were cultured at 4°C for one hour. The cells were washed, labeled by adding streptavidin-PE, allowed to stand still at 4°C for 20 minutes and analyzed by FACS CantoII (BD Biosciences) and FlowJo software (TreeStar). As shown in Figure 11, it was virtually not confirmed that TCR β-IgFc chimeric protein (TRBV16-04-Fc chimeric protein) binds to OVA peptide; however, it was confirmed that binding of TRBV16-04-Fc chimeric protein (TRBV16-04-Fc chimeric protein) to MOG peptide increases. The numerical values represent ratios (%) of positive cells.

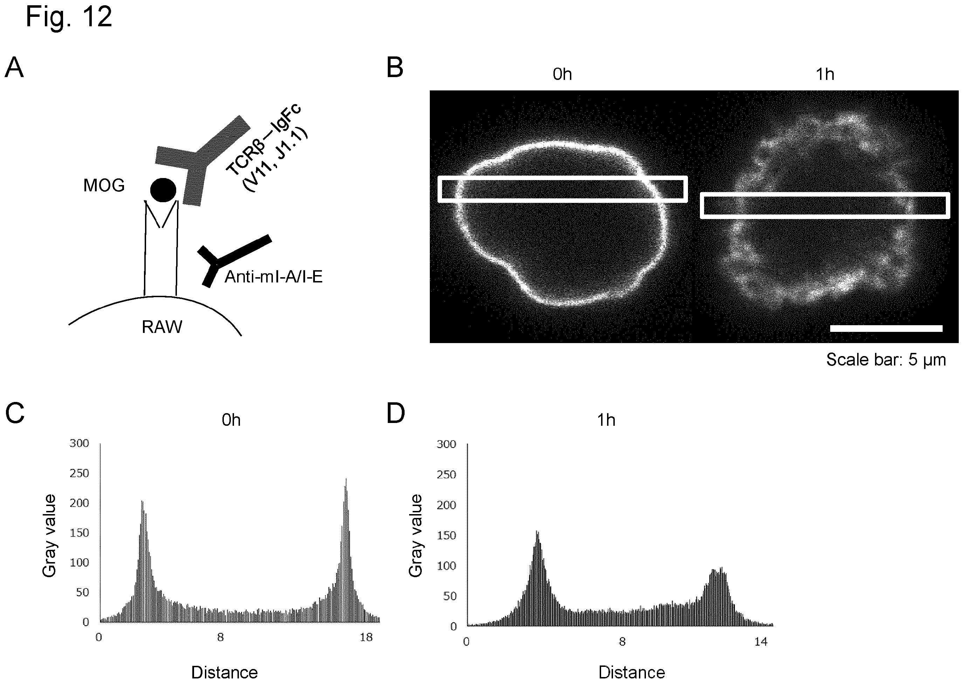

### 9. Migration of MHC class 2 molecule into cells (Figure 12)

To RAW264.7 cells (3 × 10⁵ cells), 20 µg/mL MOG peptide was added. The cells were cultured for one hour and washed. TRBV16-04-Fc chimeric protein (25µg/mL) was added and allowed to bind for one hour. The cells were washed, stained with an anti-I-A/I-E antibody (BioLegend, Clone M5/114.15.2) for one hour and labeled with MHC class II. One of the groups was directly observed (0 hr); whereas the other group was cultured at 37°C for one hour (37°C 1 hr), and thereafter, observed by a confocal microscope (Leica TCS SP8). Image analysis was performed by Image J Plot Profile (Figure 12C). Figure 12A schematically shows the process of the experiment; Figure 12B shows photographs of images observed by the confocal microscope; and Figure 12C shows the results of quantitative image analysis on confocal micrographs.

It was found that MHC class II (molecules) migrated into a cell by addition of TRBV16-04-Fc chimeric protein and demonstrated that T-cell receptor chimeric protein regulates expression of MHC class II.

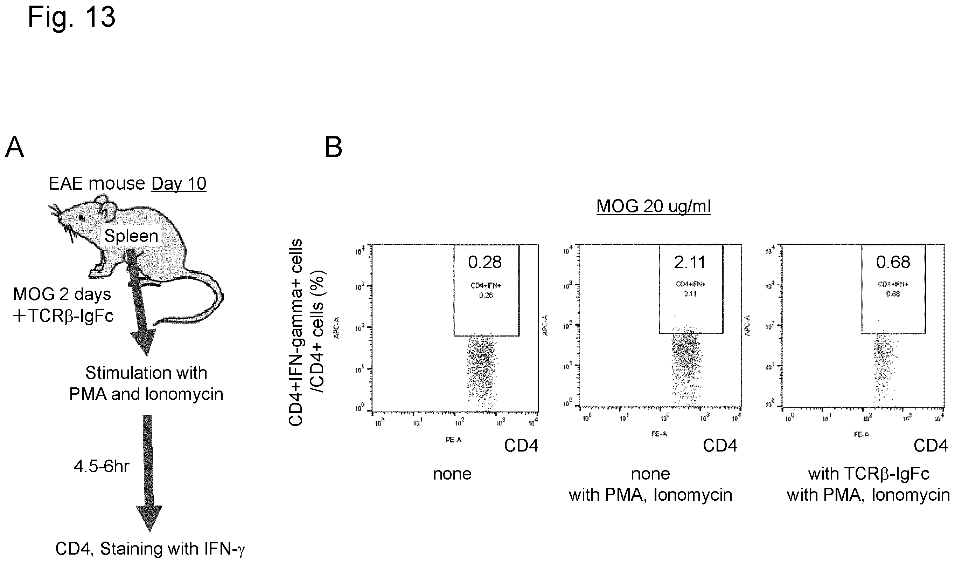

### 10. Inhibitory effect against pathogenic T cells by T-cell receptor chimeric protein in EAE model (Figure 13, Figure 14, Figure 15)

From an EAE model on Day 15, the spleen and inguinal lymph node were collected. To 2 × 10⁵ cells, 20 µg/mL MOG and 5 µg/mL T-cell receptor chimeric protein (TRBV16-04-Fc chimeric protein, TRAV9D-2-Fc chimeric protein) were added to prepare groups. Separately, a group having no additives was prepared. Both groups were cultured for 48 hours. PMA (50 ng/mL) and ionomycin (500 ng/mL) were further added to a medium and the cells were cultured for 4.5 hours. Thereafter, a cell surface was labeled with anti-CD4 antibody (BioLegend; Clone GK1.5), and the cells were suspended in a permeabilization buffer (100 µl) for 30 minutes. In this manner, a cell membrane permeabilization treatment was carried out. The interior of the cells was stained with anti-IFN-y antibody (BioLegend; Clone XMG1.2) and anti-IL-17 antibody (BioLegend; Clone B114205). Analysis was carried out by using FACS Canto II (BD Biosciences) and FlowJo software (TreeStar). Figure 13A schematically shows the process for the experiment. Figure 13B shows the ratio of IFN-γ positive CD4+ T cells. In Figure 13B, an inhibitory effect was not confirmed in the case of TRAV9D-2-Fc chimeric protein (Vα3.2Jα18-IgFc chimeric protein); however, in the case of adding TRBV16-04-Fc chimeric protein (Vβ11DJβ1.1-IgFc chimeric protein), the ratio of the IFN-γ positive CD4+ T cells decreases and an inhibitory effect was confirmed.

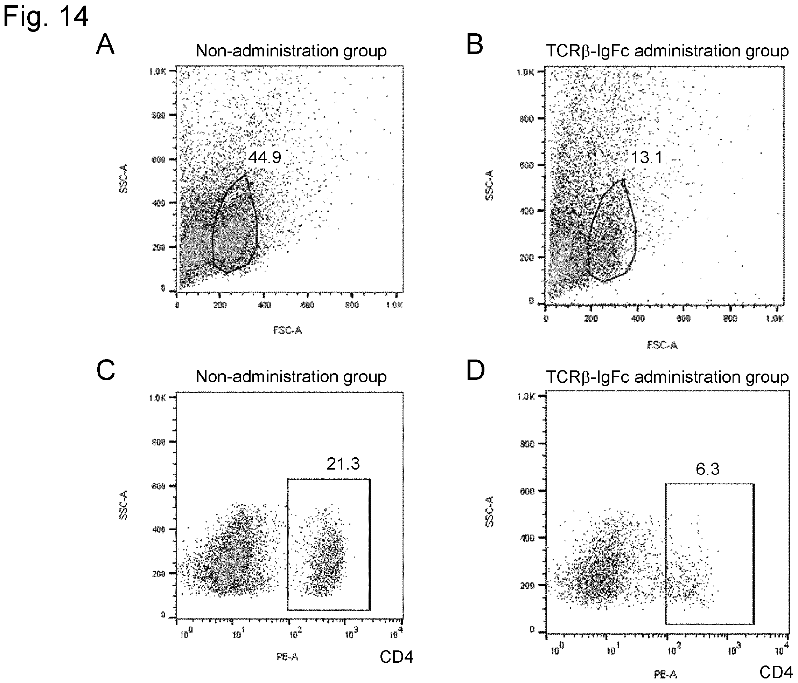

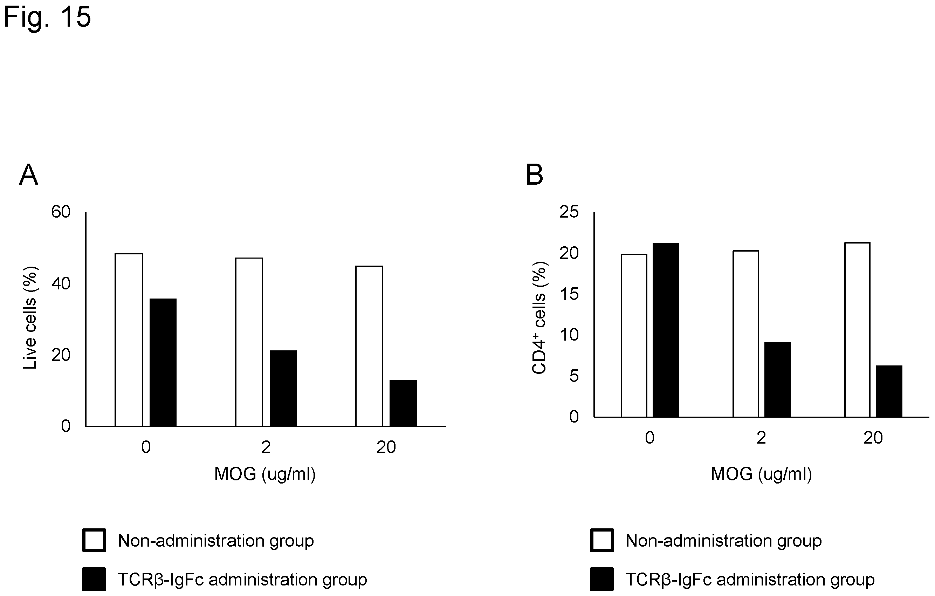

The cells were cultured in the presence of MOG (20 µg/mL) and Fc fusion protein, for 2 days, re-stimulated with PMA and ionomycin, and analyzed by flow cytometry. As a result, it was found that an inhibitory effect was not confirmed in the spleen or inguinal lymph node in the case of TRAV9D-2-Fc. In TRBV16-04-Fc chimeric protein administration group compared to the non-administered group, inhibitory effect was low (Figures 14A, B). The ratio of the CD4+ T cells remarkably decreased (Figures 14C, D). The cells cultured in the presence of MOG (0, 2, 20 µg/mL) and T-cell receptor chimeric protein (2.5 µg/mL) for 2 days, were stimulated with PMA and ionomycin and analyzed by flow cytometry. The ratio of a cell population, which was presumed to be living cells, decreased in a TRBV16-04-FcT administration group (Figure 15A) and the ratio of CD4+ T cells decreased (Figure 15B).

From these results, it was considered that TRBV16-04-Fc regulates not only competitive inhibition with pathogenic T cells but also expression of MHC and induces activation of pathogenic T cells and suppression of cell proliferation.

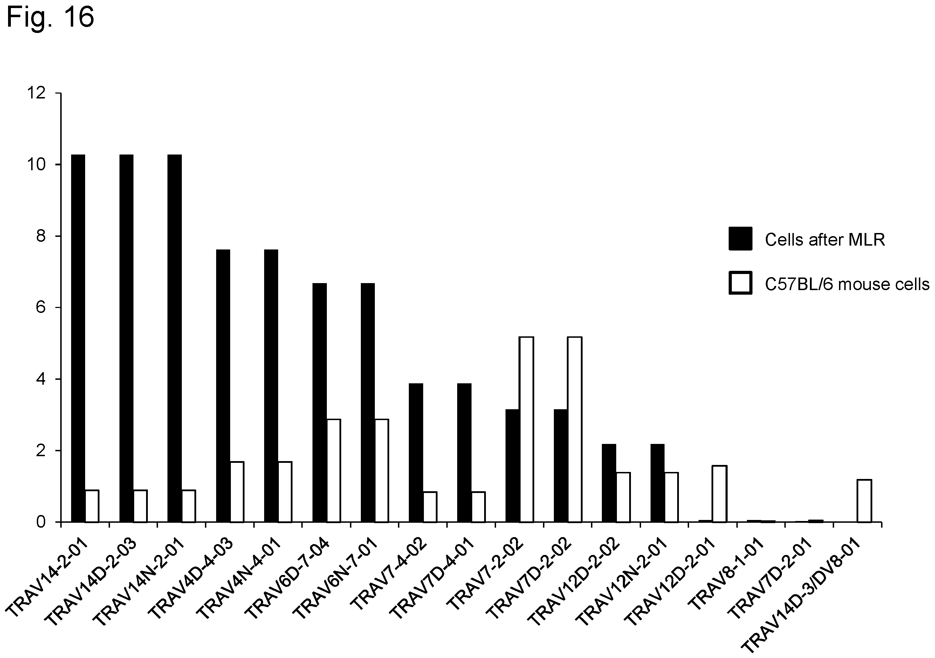

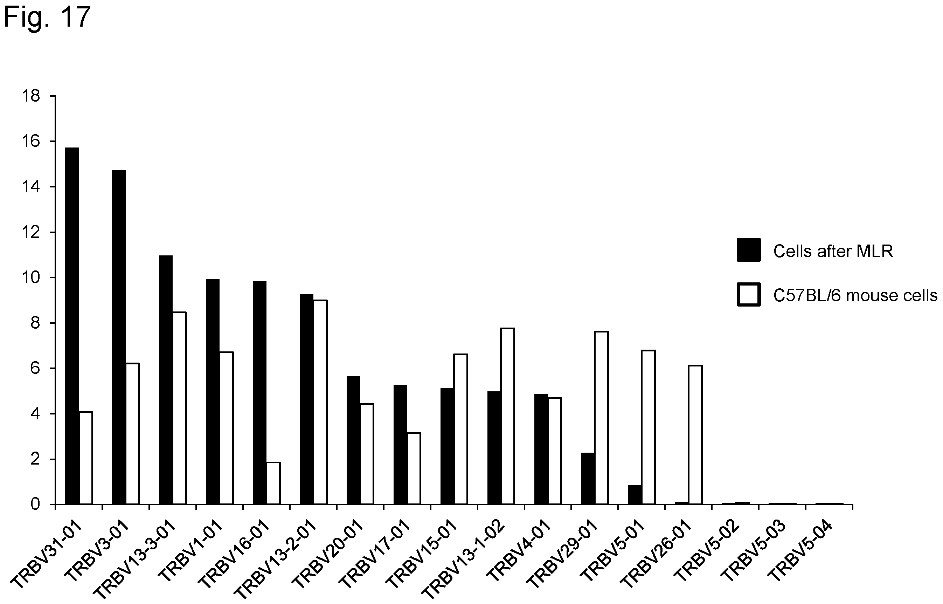

### 11. T-cell receptor (TCR) repertoire analysis for a transplant rejection model in lymphocyte mixed culture (MLR) (Figure 16, Figure 17)

Lymphocyte mixed culture was carried out in accordance with the following process. The spleen was excised out from an 8 week-old female Balb/c mouse (H-2K^{d}D^{d}). After mononuclear cells were separated and hemolysis was carried out. This was suspended in a 5% FCS/RPMI and irradiated with X ray (20 Gy) to obtain stimulator cells to be used in this experiment. Subsequently, a cell suspension was prepared from the spleen of the 8 week-old female C57BL/6 mouse (H-2K^{b}D^{b}) in the same manner to obtain responder cells. A culture was carried out in a 10% FCS-containing RPMI1640 complete medium for 7 Days at 37°C. After culturing, cells were collected and subjected to T cell repertoire analysis performed in accordance with the above process. As a control, the cells prepared from the spleen of a C57BL/6 mouse (H-2K^{b}D^{b}) were used. T-cell receptor repertoire analysis was carried out in accordance with the aforementioned process.

Figure 16 shows the results of T-cell receptor repertoire analysis of α chain performed in a lymphocyte mixed culture and Figure 17 shows the results of β chain. As shown in the figures, in either case of α chain and β chain, a plurality of TCRs reacting in MLR were successfully specified. Of the TCRs in each case, a TCR was arbitrarily selected and cloned. The cloned TCRs were as follows. TCR α chain: TRAV 7D-4-1 CDR3 AARLTGNTGKLI TRAJ 37-01 (SEQ ID NO: 8: hereinafter, TRAV7D-4-1) and TCR β chain: TRBV31-01 CDR3 AWRDWGNYAEQF TRBJ 2-1-01 (SEQ ID NO: 9: hereinafter, TRBV31-01). Using the cloned TCRs, T-cell receptor chimeric proteins were prepared in accordance with the aforementioned process.

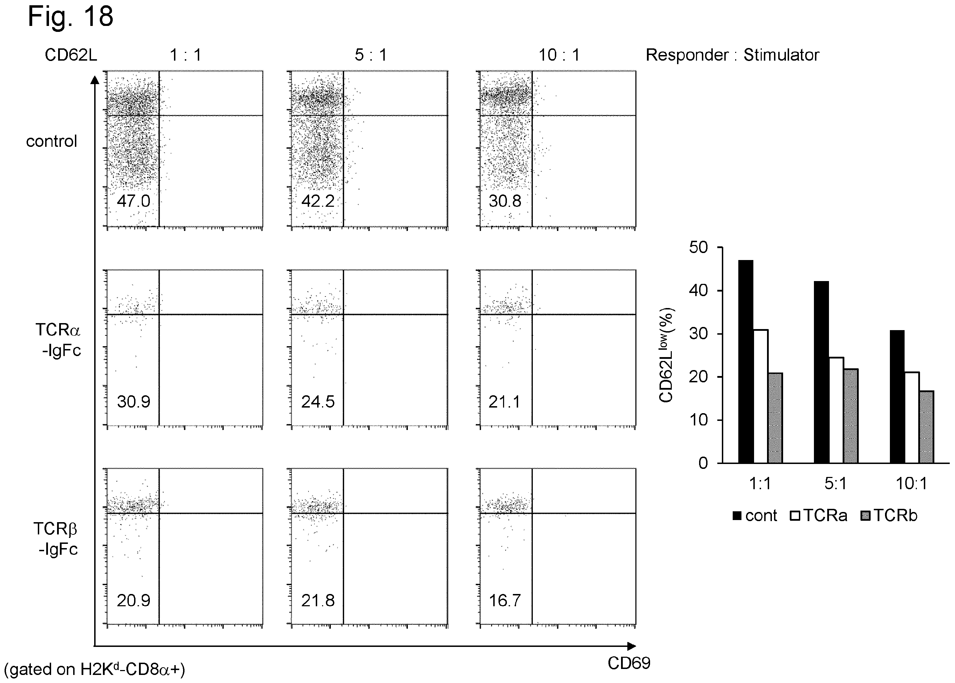

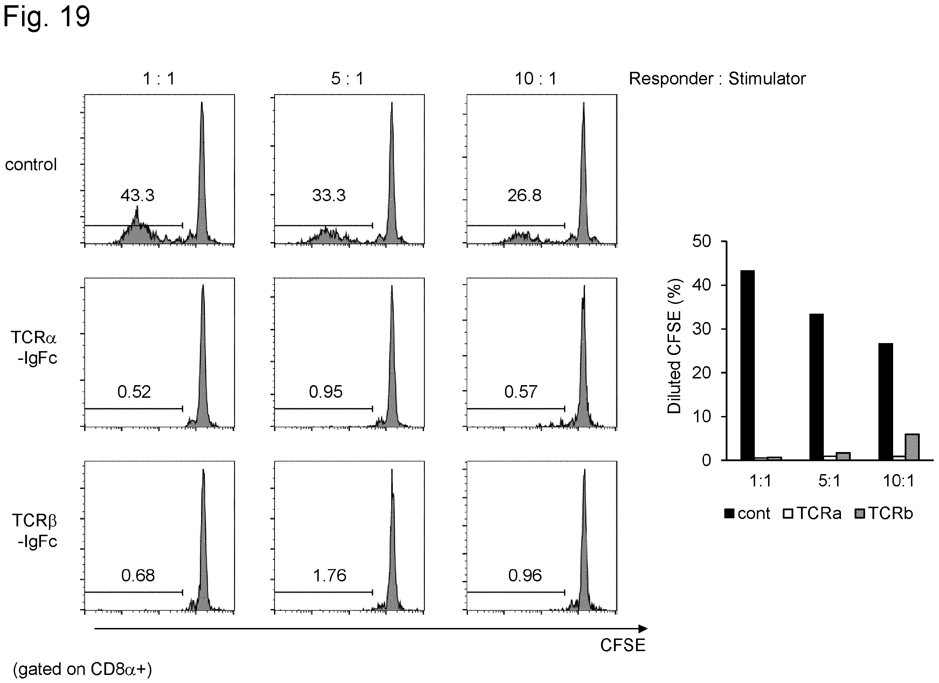

### 12. Effect of TCR-Fc on activation/proliferation of responder T cells in in-vitro MLR (Figure 18, Figure 19)

The spleen was excised out from an 8 week-old female Balb/c mouse (H-2K^{d}D^{d}) and a cell suspension was prepared in accordance with a routine method. After this was suspended in 5% FCS/RPMI, the resultant suspension was irradiated with X-ray (20 Gy) to obtain stimulator cells (S) to be used in this experiment. Subsequently, a cell suspension was prepared from the spleen of the 8 week-old female C57BL/6 mouse (H-2K^{b}D^{b}) in the same manner to obtain responder cells (R). In order to check cell proliferation, part of the cells was labelled with CFSE (5 µM).

The responder cells (R) were prepared so as to have a density of 1 × 10⁶ cells/50 µl (in 10% FCS/RPMI) and the volume of S was controlled to be 50 µl such that the ratio of R: S became 1: 1, 5: 1, 10: 1 and seeded in the wells of a 96-well U-bottom plate. TCRα (TRAV 7D-4-1 CDR3 AARLTGNTGKLI TRAJ 37-01) and β chain (TRBV31-01 CDR3 AWRDWGNYAEQF TRBJ 2-1-01), which were identified by *in-vitro* MLR, were separately introduced into pFuse mIgG2a vector (Invivogen, hereinafter, referred to as TRAV7D-4-1-Fc and TRBV31-01-Fc, respectively) by recombination technique. In this manner, T-cell receptor chimeric proteins were prepared. A culture supernatant (100 µl) containing the T-cell receptor chimeric protein was added in *in-vitro* MLR and the reaction thereof was observed. Activation of Responder cells (R) was checked by analyzing H-2K^{d}-CD8a + CD62Llow, 72 hours after culture; whereas, proliferation of the cells was checked by analyzing proliferation of CD8a + CFSElow cells, by flow cytometry.

As shown in Figure 18, in both groups containing T-cell receptor α-chain chimeric protein and T-cell receptor β chain chimeric protein, respectively, the ratio of H-2K^{d}-CD8a + CD62Llow cell populations decreased. The numerical values in the figure represent the ratios (%) of cells in respective fractions. Since H-2K^{d}-CD8a + CD62Llow cells were activated cells, a decrease of the proportion of the cells means that both of TRAV7D-4-1-Fc and TRBV31-01-Fc inhibit activation of MLR.

In Figure 19, in both of the groups containing TRAV7D-4-1-Fc and TRBV31-01-Fc, the proliferation of CD8a + CFSElow cells decreased. The numerical values in the figure represent the ratios (%) of cells in respective fractions. More specifically, it is shown that both T-cell receptor α-chain chimeric protein and T-cell receptor β chain chimeric protein inhibit proliferation of activated cells in MLR.

MLR is an *in-vitro* simplified experiment system for organ transplant rejection, in which T cells to donor MHC are regarded as pathogenic T cells mainly causing organ transplant rejection. Accordingly, it was considered that T-cell receptor α-chain chimeric protein and T-cell receptor β chain chimeric protein bind to a donor MHC and not only cause competitive inhibition with pathogenic T cells but also inhibit activation and proliferation of pathogenic T cells by regulating expression of the donor MHC.

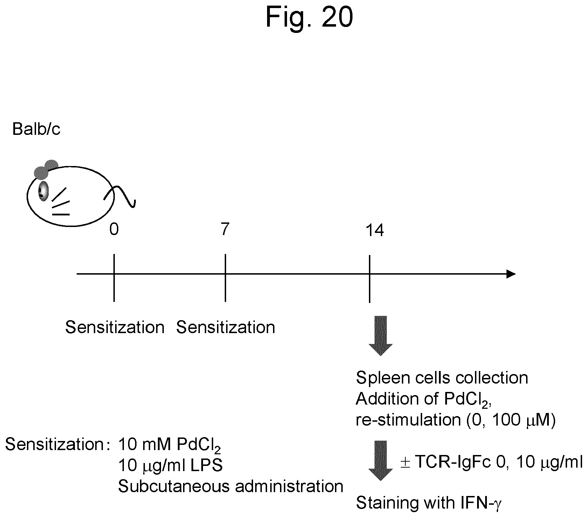

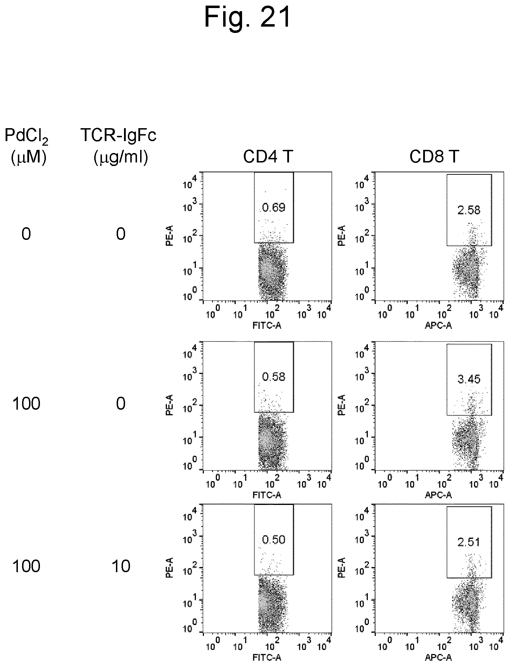

### 13. Inhibitory effect of activation T cells by TCR-IgFc in metallic allergy (Figure 20, Figure 21)

To a metallic allergy mouse model, sensitization and *in-vitro* re-stimulation were applied, as shown in Figure 20. On Day 0 and Day 7, 125 µl of a mixed solution of 10 mM PdCl₂ and 10 µg/mL LPS was subcutaneously administered to each of both inguinal parts. In this manner, sensitization was carried out. Seven days after the second sensitization, the spleen was taken out and 1 × 10⁶ (spleen) cells were re-stimulated with 0 and 100 µM PdCl₂ for 16 hours. A chimeric protein of TCR α chain (TRAV8-1-01-CDR3 (ATLYSGGSNAKLT)-TRAJ42-01) and mIgG2aFc region identified in palladium allergy was added in an amount of 0 and 10 µg/mL in some of the wells. Thereafter, the interior of the cells was stained and IFN-γ was measured by flow cytometry.

In Figure 21, it is found that CD8T cells increase by addition of 100 µM PdCl₂; however, the increase of the cells was suppressed by adding TCR α chain chimeric protein, which means that activation/proliferation of pathogenic T cells in metallic allergy can be suppressed by T cell chimeric protein. It is considered that metallic allergy is caused since a metallic allergy substance is presented as an antigen in MHC. It was considered that T cell chimeric protein causes not only competitive inhibition with pathogenic T cells but also regulates expression of MHC, thereby inhibiting activation and proliferation of pathogenic T cells.

### [Example 2]

### 1. Experiment for EAE treatment

### (1) Construction of soluble TCR and purification of protein

MOG-reactive TCRs (2D2 TCR) (TRAV9-2D-2 + CDR3 (VYFCALRSYNFG (SEQ ID NO: 10)) + TRAJ23, TRBV16-04 + CDR3 (CASSLDCGANP (SEQ ID NO: 11)) + TRBJ1-1-01) were respectively introduced in human IgG Hole/pcDNA3.4 and human IgG Knob/pcDNA3.4 by HiFi Assembly in accordance with recombination technique. These two plasmids were introduced in Expi293F by lipofection and a soluble heterodimer, i.e., TCR α-TCRβ, was produced by "knob-into-hole". Culture was carried out in the conditions of 37°C, 8% CO₂ and 125 rpm for 7 days and the culture supernatant was passed through Protein G Sepharose (GE Healthcare) column to purify TCR-Fc fusion protein (2D2 TCRαβ-Fc).

### (2) Preparation of experimental autoimmune encephalomyelitis (EAE) model and experiment for EAE treatment

To 6-8 week-old female mice, C57BL/6J, 200 µg of myelin oligodendrocyte glycoprotein (MOG) 35-55 peptide (MOG: MEVGWYRSPFSRVVHLYRNGK) (SEQ ID NO: 7) (company: ANASPEC), which was suspended in complete Freund's adjuvant (CFA) containing 5 mg/mL *mycobacterium tuberculosis* H37Ra dead cells, was subcutaneously administered 10 days before and Day 0. On the same day as MOG peptide subcutaneous administration and 2 days later, 200 ng of pertussis toxin (BD Difco) was intraperitoneally administered to immunize the mice. Fc fusion protein was subcutaneously administered 4 hours before the MOG peptide administration. PBS was administered as a control (cont). The onset was evaluated based on the clinical score indicating the degree of paralysis (0: asymptomatic, 1: loss of tension in tail, 2: paralysis of single lower limb, 3: paralysis of both lower limbs, 4: cannot walk by itself, 5: lethal).

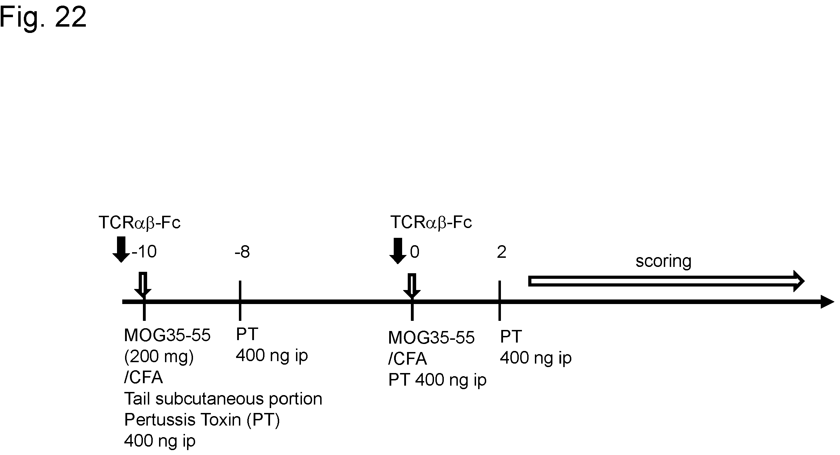

The process of the experiment is schematically shown in Figure 22.

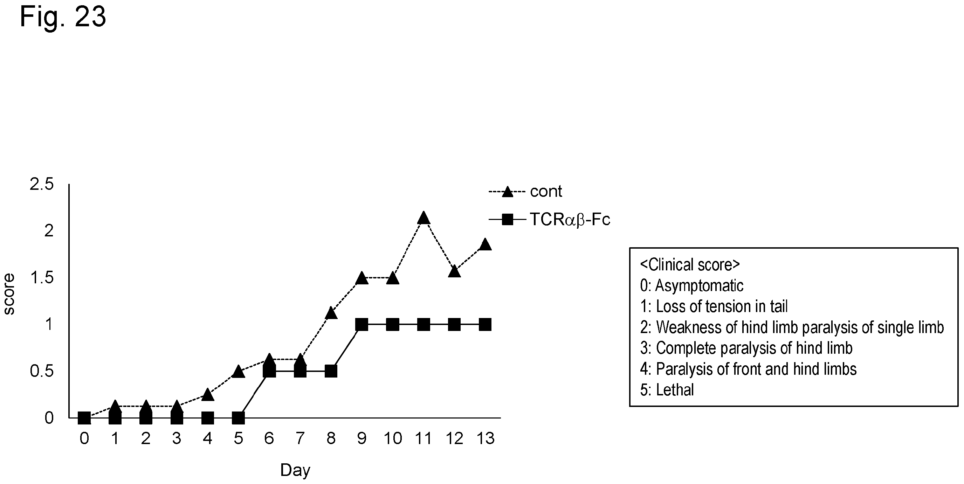

The results are shown in Figure 23. As shown in Figure 23, when an Fc fusion protein (TCR-Fc 2D2 TCRαβ-Fc) was administered, progression of symptom was successfully suppressed.

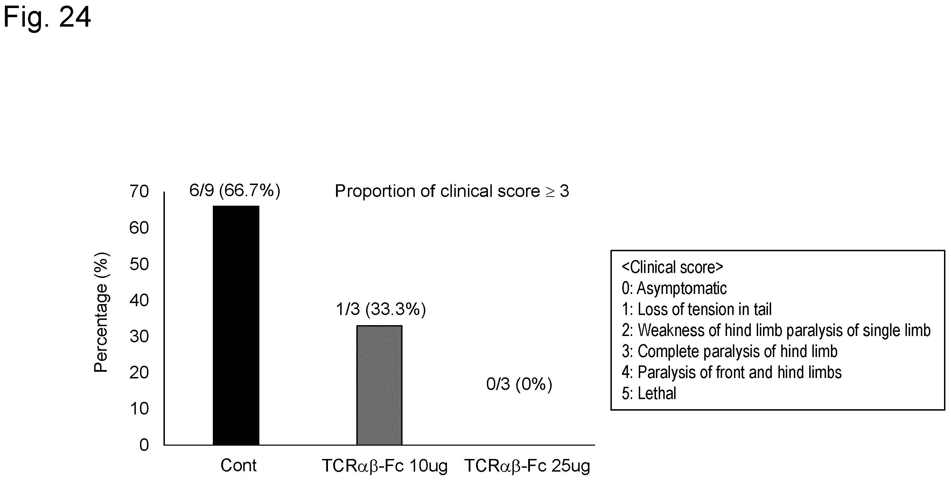

The ratio of mice whose clinical score reached 3 or more until Day 17 is shown in Figure 24. The ratio of the mice whose clinical score reached 3 or more until Day 17 was 66.7% (6/9) in a Fc protein non-administration group (w/o Fc-protein). The ratio in a TCR-Fc (2D2 TCRαβ-Fc) 10 µg × 2 administration group was 33.3% (1/3) and that in a TCR-Fc (2D2 TCRαβ-Fc) 25 µg × 2 administration group was 0% (0/3). From the results, it was found that the score significantly decreased, up to 3 or more, by administration of TCR-Fc. More specifically, serious progression of the symptom of EAE was suppressed by administration of 2D2 TCRαβ-Fc. From the above, it was demonstrated that 2D2 TCRαβ-Fc not only suppresses progress of the EAE symptom but also produces an effect on suppression of serious progression of EAE symptom.

### 2. Experiment for metallic allergy treatment

### (1) Construction of soluble TCR-Fc and purification of protein

TCRα (TRAV7-2-02 + CDR3 (AATSSGSWQLI (SEQ ID NO: 12)) + TRAJ22-01) elevated by induction of palladium allergy in C57BL/6 mice was introduced in a pFuse mIgG2a vector (Invivogen) by a recombination technique to construct a plasmid of a fusion protein with mIgG2a (mTRAV7-Fc/pFuse). The plasmid was introduced into Expi293F cells (Thermo Fisher Sciences) by lipofection. Thereafter, culture was carried out in the conditions of 37°C, 8% CO₂ and 125 rpm for 7 days. The culture supernatant was passed through Protein G Sepharose (GE Healthcare) column to purify Fc fusion protein.

### (2) Preparation of metallic allergy model mouse and experiment for treatment with soluble TCR-Fc administration

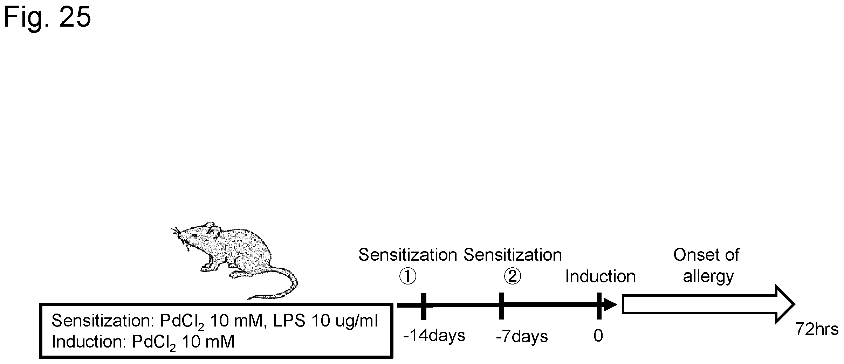

On Day 0 and Day 7, 125 µl of a mixed solution of 10 mM PdCl₂ and 10 µg/mL LPS was subcutaneously administered to each of both inguinal parts of wild-type C57BL/6 mice. In this manner, sensitization of the mice was carried out. Seven days after sensitization, 25 µl of PdCl₂ (10 mM) was subcutaneously administered to a hind-limb footpad to induce allergy. The thickness of the footpad was measured at the intervals of 24 hours. Soluble TCR was intraperitoneally administered one hour before sensitization and (allergy) induction; more specifically, mTRAV7-Fc and mIgGFc (cont) (each 10 mM) and a positive control, i.e., 1 mg/Kg olopatadine (OLP), which is an antihistamine, were intraperitoneally administered. The process for the experiment is schematically shown in Figure 25.

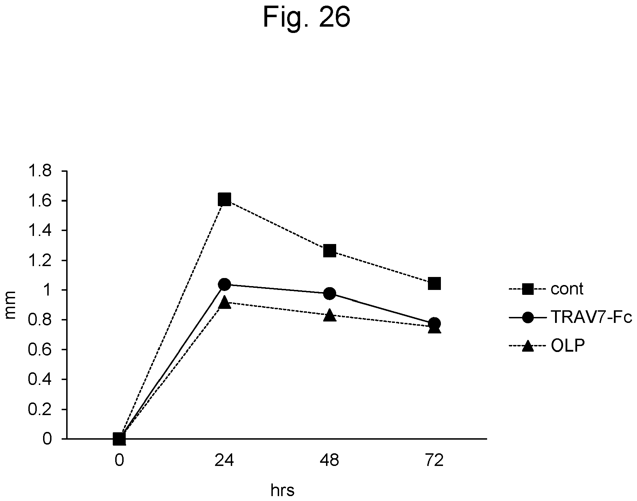

The results are shown in Figure 26. As shown in Figure 26, when a TCR-Fc fusion protein (TRAV7-Fc) was administered, the hypertrophy of footpad was suppressed to the same level as in the positive control. Accordingly, it was demonstrated that TCR-Fc has the same effect as that of an antihistamine drug (an existing drug) on metallic allergy.

### Industrial Applicability

The T-cell receptor chimeric protein of the present invention can be used in treatment and detection of an autoimmune disease, organ transplant rejection and allergic disease.

### Sequence listing free text

SEQ ID NO: 1 to 4, 7 to 12 synthesis
SEQ ID NO: 5, 6 primer

All the publications, the patents and patent applications cited in the specification are incorporated herein in their entireties by reference.

## Claims

1. An agent for down-modulating MHC molecular complex comprising, as an active ingredient, a T-cell receptor chimeric protein being a fusion protein of a T-cell receptor variable region recognizing a complex of an autoimmune disease-specific antigen and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex, in an antigen specific manner.

2. An agent for down-modulating MHC molecular complex comprising, as an active ingredient, a T-cell receptor chimeric protein being a fusion protein of a T-cell receptor variable region recognizing a complex of a donor-derived antigen specific to an organ transplant rejection and an MHC molecule and an immunoglobulin Fc region, wherein the agent binds to a complex of the donor-derived specific antigen and the MHC molecule on an antigen-presenting cell or a target cell recognized by a pathogenic T cell to reduce the expression of the MHC molecular complex, in an antigen specific manner.

3. An agent for down-modulating MHC molecular complex comprising, as an active ingredient, a T-cell receptor chimeric protein being a fusion protein of a T-cell receptor variable region recognizing a complex of a specific antigen to an allergic disease and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex, in an antigen specific manner.

4. The agent for down-modulating MHC molecular complex according to any one of claims 1 to 3, wherein the T-cell receptor chimeric protein comprises a variable region, whole of CDR3 and J region of a T-cell receptor.

5. The agent for down-modulating MHC molecular complex according to any one of claims 1 to 4, wherein the T-cell receptor variable region is α chain and/or β chain, or γ chain and/or δ chain of the T-cell receptor.

6. The agent for down-modulating MHC molecular complex according to any one of claims 1 to 5, wherein the immunoglobulin Fc region is an Fc region of IgG.

7. The agent for down-modulating MHC molecular complex according to any one of claims 1 to 6, wherein the agent is a dimer of two fusion proteins of the T-cell receptor variable region and the immunoglobulin Fc region and the two proteins are bonded to each other by disulfide bond.

8. The agent for down-modulating MHC molecular complex according to any one of claims 1 to 7, wherein the T-cell receptor binds to the complex of the antigen and the MHC molecule.

9. The agent for down-modulating MHC molecular complex according to any one of claims 1 to 8, wherein the MHC molecule is a classical MHC molecule or a non-classical MHC molecule.

10. An agent for decreasing T-cell function causing an autoimmune disease, comprising, as an active ingredient, a T-cell receptor chimeric protein being a fusion protein of a T-cell receptor variable region recognizing a complex of an autoimmune disease-specific antigen and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex in order to avoid recognition by the pathogenic T cell.

11. An agent for decreasing T-cell function for decreasing T-cell function causing organ transplant rejection, comprising, as an active ingredient, a T-cell receptor chimeric protein being a fusion protein of a T-cell receptor variable region recognizing a complex of a donor-derived antigen specific to an organ transplant rejection and an MHC molecule and an immunoglobulin Fc region, wherein the agent binds to a complex of the donor-derived specific antigen and the MHC molecule on an antigen-presenting cell or a target cell recognized by a pathogenic T cell to reduce the expression of the MHC molecular complex in order to avoid recognition by the pathogenic T cell.

12. An agent for decreasing T-cell function causing an allergic disease, comprising, as an active ingredient, a T-cell receptor chimeric protein being a fusion protein of a T-cell receptor variable region recognizing a complex of an allergic disease-specific antigen and an MHC molecule and an immunoglobulin Fc region, wherein the agent binds to a complex of the specific antigen and the MHC molecule on an antigen-presenting cell or a target cell recognized by a pathogenic T cell to reduce the expression of the MHC molecular complex in order to avoid recognition by the pathogenic T cell.

13. The agent for decreasing T-cell function according to any one of claims 10 to 12, wherein the T-cell receptor chimeric protein comprises a variable region, whole of CDR3 and J region of a T-cell receptor.

14. The agent for decreasing T-cell function according to any one of claims 10 to 13, wherein the T-cell receptor variable region is α chain and/or β chain, or γ chain and/or δ chain of the T-cell receptor.

15. The agent for decreasing T-cell function according to any one of claims 10 to 14, wherein the immunoglobulin Fc region is Fc region of IgG.

16. The agent for decreasing T-cell function according to any one of claims 10 to 15, wherein the agent is a dimer of two fusion proteins of the T-cell receptor variable region and the immunoglobulin Fc region, wherein the two proteins are bonded to each other by disulfide bond.

17. The agent for decreasing T-cell function according to any one of claims 10 to 16, wherein the T-cell receptor binds to the complex of the antigen and the MHC molecule.

18. The agent for decreasing T-cell function according to any one of claims 10 to 17, wherein the MHC molecule is a classical MHC molecule or a non-classical MHC molecule.

19. A therapeutic agent for an autoimmune disease, comprising the agent for down-modulating the MHC molecular complex according to any one of claims 1 and 4 to 9.

20. An agent for suppressing an organ transplant rejection, comprising the agent for down-modulating the MHC molecular complex according to any one of claims 2 and 4 to 9.

21. A therapeutic agent for an allergic disease, comprising the agent for down-modulating the MHC molecular complex according to any one of claims 3 to 9.

22. A therapeutic agent for an autoimmune disease, comprising the agent for decreasing T-cell function according to any one of claims 10 and 13 to 18.

23. An agent for suppressing an organ transplant rejection, comprising the agent for decreasing T-cell function according to any one of claims 11 and 13 to 18.

24. A therapeutic agent for an allergic disease, comprising the agent for decreasing T-cell function according to any one of claims 12 to 18.

25. A method for detecting an autoimmune disease, comprising the steps of:
bringing a labeled T-cell receptor chimeric protein being a fusion protein of a T-cell receptor variable region recognizing a complex of an autoimmune disease-specific antigen and an MHC molecule and an immunoglobulin Fc region, into contact with cells taken from a biological sample of a test subject; and
determining that a target cell is present in the test subject and the test subject has an autoimmune disease if the T-cell receptor chimeric protein binds to a cell taken from the biological sample of the test subject.

26. The method for detecting an autoimmune disease according to claim 25, wherein the MHC molecule is a classical MHC molecule or a non-classical MHC molecule.

27. A reagent for detecting an autoimmune disease, comprising a labeled T-cell receptor chimeric protein being a fusion protein of a T-cell receptor variable region recognizing a complex of an autoimmune disease-specific antigen and an MHC molecule and an immunoglobulin Fc region.

28. The reagent for detecting an autoimmune disease, according to claim 27, wherein the MHC molecule is a classical MHC molecule or a non-classical MHC molecule.

29. A method for detecting a rejection causing organ transplant rejection, comprising the steps of:
bringing a labeled T-cell receptor chimeric protein being a fusion protein of a T-cell receptor variable region recognizing a complex of a donor-derived antigen specific to organ transplant rejection and an MHC molecule and an immunoglobulin Fc region, into contact with cells taken from a biological sample of a test subject; and
determining that a target cell is present in the test subject and a rejection occurs in the test subject if the T-cell receptor chimeric protein binds to a cell taken from the biological sample of the test subject.

30. The method for detecting a rejection according to claim 29, wherein the MHC molecule is a classical MHC molecule or a non-classical MHC molecule.

31. A reagent for detecting a rejection, comprising a labelled T-cell receptor chimeric protein being a fusion protein of a T-cell receptor variable region recognizing a complex of an organ transplant rejection-specific donor-derived antigen and the MHC molecule and an immunoglobulin Fc region.

32. The reagent for detecting a rejection according to claim 31, wherein the MHC molecule is a classical MHC molecule or a non-classical MHC molecule.

33. A method for detecting an allergic disease, comprising the steps of:
bringing a labeled T-cell receptor or chimeric protein being a fusion protein of a T-cell receptor variable region recognizing a complex of an allergic disease-specific antigen and an MHC molecule and an immunoglobulin Fc region, into contact with cells taken from a biological sample of a test subject; and
determining that a target cell is present in the test subject and the test subject has an allergic disease if the T-cell receptor or chimeric protein binds to a cell taken from the biological sample of the test subject.

34. The method for detecting an allergic disease according to claim 33, wherein the MHC molecule is a classical MHC molecule or a non-classical MHC molecule.

35. A reagent for detecting an allergic disease, comprising a labeled T-cell receptor or chimeric protein being a fusion protein of a T-cell receptor variable region recognizing a complex of an allergic disease-specific antigen and an MHC molecule and an immunoglobulin Fc region.

36. The reagent for detecting an allergic disease according to claim 35, wherein the MHC molecule is a classical MHC molecule or a non-classical MHC molecule.


SEQUENCE LISTING

<110>  TOHOKU UNIVERSITY

<120>  A method of antigen-specific modulating of the expression of MHC

<130>  PH-7759-PCT

<150>  JP 2018-015908
<151>  2018-01-31

<160>  12

<170>  PatentIn version 3.5

<210>  1
<211>  8
<212>  PRT
<213>  Artificial

<220>
<223>  Synthetic

<400>  1

Ser Ile Ile Asn Phe Glu Lys Leu
1               5


<210>  2
<211>  9
<212>  PRT
<213>  Artificial

<220>
<223>  Synthetic

<400>  2

Ala Ala Ser Asp Asn Tyr Gln Leu Ile
1               5


<210>  3
<211>  12
<212>  PRT
<213>  Artificial

<220>
<223>  Synthetic

<400>  3

Ala Ala Ser Pro Val Asp Ser Asn Tyr Gln Leu Ile
1               5                   10


<210>  4
<211>  9
<212>  PRT
<213>  Artificial

<220>

<223>  Synthetic

<400>  4

Ala Ala Gly Ser Asn Tyr Gln Leu Ile
1               5


<210>  5
<211>  29
<212>  DNA
<213>  Artificial

<220>
<223>  Primer

<400>  5
gagaattcgc agcagcaggt gagacaaag                                         29


<210>  6
<211>  42
<212>  DNA
<213>  Artificial

<220>
<223>  Primer

<400>  6
gatagatctt tctgggttct ggatgtctgg ctttataatt ag                          42


<210>  7
<211>  21
<212>  PRT
<213>  Artificial

<220>
<223>  Synthetic

<400>  7

Met Glu Val Gly Trp Tyr Arg Ser Pro Phe Ser Arg Val Val His Leu
1               5                   10                  15


Tyr Arg Asn Gly Lys
            20


<210>  8
<211>  12
<212>  PRT
<213>  Artificial

<220>
<223>  Synthetic

<400>  8

Ala Ala Arg Leu Thr Gly Asn Thr Gly Lys Leu Ile
1               5                   10

<210>  9
<211>  12
<212>  PRT
<213>  Artificial

<220>
<223>  Synthetic

<400>  9

Ala Trp Arg Asp Trp Gly Asn Tyr Ala Glu Gln Phe
1               5                   10


<210>  10
<211>  12
<212>  PRT
<213>  Artificial

<220>
<223>  Synthetic

<400>  10

Val Tyr Phe Cys Ala Leu Arg Ser Tyr Asn Phe Gly
1               5                   10


<210>  11
<211>  11
<212>  PRT
<213>  Artificial

<220>
<223>  Synthetic

<400>  11

Cys Ala Ser Ser Leu Asp Cys Gly Ala Asn Pro
1               5                   10


<210>  12
<211>  11
<212>  PRT
<213>  Artificial

<220>
<223>  Synthetic

<400>  12

Ala Ala Thr Ser Ser Gly Ser Trp Gln Leu Ile
1               5                   10

SEQUENCE LISTING

<110>  TOHOKU UNIVERSITY

<120>  Method for Regulating Antigen-Specific MHC Expression

<130>  007703705

<140>  EP 19746998.4
<141>  2019-01-31

<150>  PCT/JP2019/003363
<150>  2019-01-31

<150>  JP 2018-015908
<151>  2018-01-31

<160>  12

<170>  PatentIn version 3.5

<210>  1
<211>  8
<212>  PRT
<213>  Artificial

<220>
<223>  Synthetic

<400>  1

Ser Ile Ile Asn Phe Glu Lys Leu
1               5

<210>  2
<211>  9
<212>  PRT
<213>  Artificial

<220>
<223>  Synthetic

<400>  2

Ala Ala Ser Asp Asn Tyr Gln Leu Ile
1               5

<210>  3
<211>  12
<212>  PRT
<213>  Artificial

<220>
<223>  Synthetic

<400>  3

Ala Ala Ser Pro Val Asp Ser Asn Tyr Gln Leu Ile
1               5                   10

<210>  4
<211>  9
<212>  PRT
<213>  Artificial

<220>
<223>  Synthetic

<400>  4

Ala Ala Gly Ser Asn Tyr Gln Leu Ile
1               5


<210>  5
<211>  29
<212>  DNA
<213>  Artificial

<220>
<223>  Primer

<400>  5
gagaattcgc agcagcaggt gagacaaag                                         29


<210>  6
<211>  42
<212>  DNA
<213>  Artificial

<220>
<223>  Primer

<400>  6
gatagatctt tctgggttct ggatgtctgg ctttataatt ag                          42


<210>  7
<211>  21
<212>  PRT
<213>  Artificial

<220>
<223>  Synthetic

<400>  7

Met Glu Val Gly Trp Tyr Arg Ser Pro Phe Ser Arg Val Val His Leu
1               5                   10                  15


Tyr Arg Asn Gly Lys
            20


<210>  8
<211>  12
<212>  PRT
<213>  Artificial

<220>
<223>  Synthetic

<400>  8

Ala Ala Arg Leu Thr Gly Asn Thr Gly Lys Leu Ile
1               5                   10


<210>  9
<211>  12
<212>  PRT
<213>  Artificial

<220>
<223>  Synthetic

<400>  9

Ala Trp Arg Asp Trp Gly Asn Tyr Ala Glu Gln Phe
1               5                   10


<210>  10
<211>  12
<212>  PRT
<213>  Artificial

<220>
<223>  Synthetic

<400>  10

Val Tyr Phe Cys Ala Leu Arg Ser Tyr Asn Phe Gly
1               5                   10


<210>  11
<211>  11
<212>  PRT
<213>  Artificial

<220>
<223>  Synthetic

<400>  11

Cys Ala Ser Ser Leu Asp Cys Gly Ala Asn Pro
1               5                   10


<210>  12
<211>  11
<212>  PRT
<213>  Artificial

<220>
<223>  Synthetic

<400>  12

Ala Ala Thr Ser Ser Gly Ser Trp Gln Leu Ile
1               5                   10